# EUROPEAN PATENT APPLICATION

(11) **EP 4 349 374 A1**
(43) Date of publication of application: **10.04.2024**
(21) Application number: 22199885.9
(22) Date of filing: 05.10.2022
(51) Int. Cl.: A61K 51/00, A61P 35/00, C07K 16/28

(54) **ANTI-UROKINASE PLASMINOGEN ACTIVATOR RECEPTOR IMMUNOGLOBULIN SINGLE VARIABLE DOMAINS**

(71) Applicant: Vrije Universiteit Brussel, 1050 Elsene (BE)
(72) Inventor: The designation of the inventor has not yet been filed
(74) Representative: Brantsandpatents bv

(57) **Abstract**

The current invention relates to an immunoglobulin single variable domain that is directed against and/or that specifically binds to human urokinase plasminogen activator receptor (human uPAR, SEQ ID NO: 1), wherein the immunoglobulin single variable domain comprises polypeptides that have at least 90% amino acid sequence identity with SEQ ID NO: 2. The current invention further relates to a polypeptide comprising aforementioned immunoglobulin single variable domain, a nucleic acid sequence encoding aforementioned immunoglobulin single variable domain, a pharmaceutical composition comprising aforementioned immunoglobulin single variable domain or aforementioned polypeptide, the use of aforementioned immunoglobulin single variable domain and a method for producing aforementioned immunoglobulin single variable domain.

## Description

### FlELD OF THE INVENTION

The present invention relates to immunoglobulin single variable domains directed against human urokinase plasminogen activator receptor (uPAR). More specifically, it concerns immunoglobulin single variable domains against human uPAR, showing cross-reactivity against canine uPAR, and their use in targeting and *in vivo* imaging of tumor-associated cells, with applications in the field of cancer diagnostics and therapeutics and monitoring of the disease.

### BACKGROUND

Molecular imaging has been defined as "the visualization, characterization, and measurement of biological processes at the molecular and cellular level in living subjects". The technique makes use of molecular tracers seeking for biomarkers expressed in (patho)physiological processes. Molecular tracers most often consist of a targeting moiety to direct the tracer and a signaling moiety for detection. Following administration, the molecular tracer will specifically accumulate in areas where the biomarker reveals itself, while unbound tracer will be eliminated. The bound molecular tracer is then visualized by means of an appropriate imaging modality. Radioactivity and fluorescence are particularly suited for the application because of their high detection sensitivity. Hence, the same principle can be applied in molecular therapy for the targeted delivery of toxic substances such as drugs or therapeutic radiation.

The urokinase plasminogen activator receptor (uPAR) is a molecular target with increased expression in many human tumors, both on the cancer cells themselves as well as on stromal cells. Given the involvement of the uPAR-uPA interaction in extracellular matrix degradation, uPAR plays an important role in tumor progression and metastasis. Hence, PET imaging of uPAR overexpression has been proposed for the prediction of poor prognosis and cancer aggressiveness. Moreover, the paramount presence of uPAR on the invasive front of the tumor makes this receptor especially attractive for intraoperative fluorescence imaging.

Due to the importance of uPAR in cancer invasion and metastasis, a number of targeting agents have been identified during the last decades. The application field of uPAR-targeting agents is high, because the uPAR targeting agents can potentially be used to target multiple cancers. However, uPAR-targeting agents currently under development, such as uPAR-targeting peptides, indicate a less favourable pharmacokinetic profile.

In addition, there is strong evidence for species specificity of uPAR, showing for instance that mouse uPA (more specifically, the amino-terminal fragment (ATF) thereof) has a significantly reduced binding to human uPAR, complicating the use of xenograft mouse models.

As such, there is a need for novel uPAR-targeting agents which show an optimized pharmacokinetic profile and for which the biodistribution data can be easily extrapolated from preclinical animal models to human.

The present invention aims to resolve at least some of the problems and disadvantages mentioned above.

### SUMMARY OF THE INVENTION

The present invention and embodiments thereof serve to provide a solution to one or more of above-mentioned disadvantages. To this end, the present invention relates to an immunoglobulin single variable domain according to claim 1. Said immunoglobulin single variable domain is directed against and/or specifically binds to human urokinase plasminogen activator receptor (human uPAR, SEQ ID NO: 1), wherein the immunoglobulin single variable domain comprises polypeptides that have at least 90% amino acid sequence identity with SEQ ID NO: 2.

The inventors surprisingly found that the immunoglobulin single variable domains of the invention possess an especially favourable *in vivo* biodistribution profile, including rapid pharmacokinetics, with rapid blood clearance and elimination through the kidneys, fast and homogenous tumor uptake, and low non-specific uptake in organs with no uPAR expression. Furthermore, the immunoglobulin single variable domain of the invention shows cross-reactivity with canine (dog) urokinase plasminogen activator receptor (canine/dog uPAR, SEQ ID NO: 10), enabling to better assess the biodistribution and other characteristics of the immunoglobulin single variable domain making use of more relevant larger animal models with spontaneous tumors.

Preferred embodiments of the immunoglobulin single variable domain of the invention are shown in any of the claims 2 to 9.

In a second aspect, the present invention relates to a polypeptide according to claim 10, said polypeptide comprising aforementioned immunoglobulin single variable domain.

In a third aspect, the present invention relates to a nucleic acid sequence according to claim 11, said nucleic acid encoding aforementioned immunoglobulin single variable domain or polypeptide.

In a fourth aspect, the present invention relates to a pharmaceutical composition according to claim 12, said composition comprising aforementioned immunoglobulin single variable domain or polypeptide and optionally at least one of a pharmaceutically acceptable carrier, adjuvant or diluent.

In further aspects, the present invention relates to use of aforementioned immunoglobulin single variable domain or polypeptide, according to claims 13-14.

### DESCRIPTION OF FIGURES

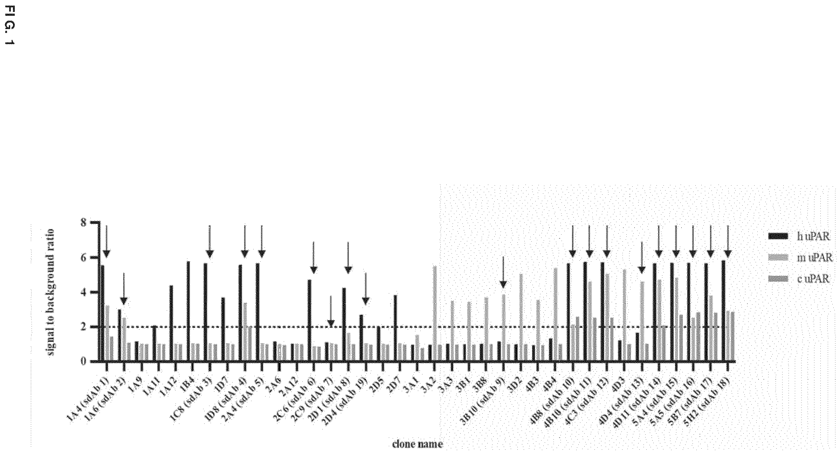

**Figure 1** shows a graphical representation of BE-ELISA results for the 37 preselected clones identified as described in example 1, showing the ratio of measured signal in positive and control conditions for h, m and c uPAR, respectively. Arrows indicate the clones selected for further *in vitro* evaluation, including single domain Antibody (sdAb) 15, an immunoglobulin single variable domain according to an embodiment of the present invention.

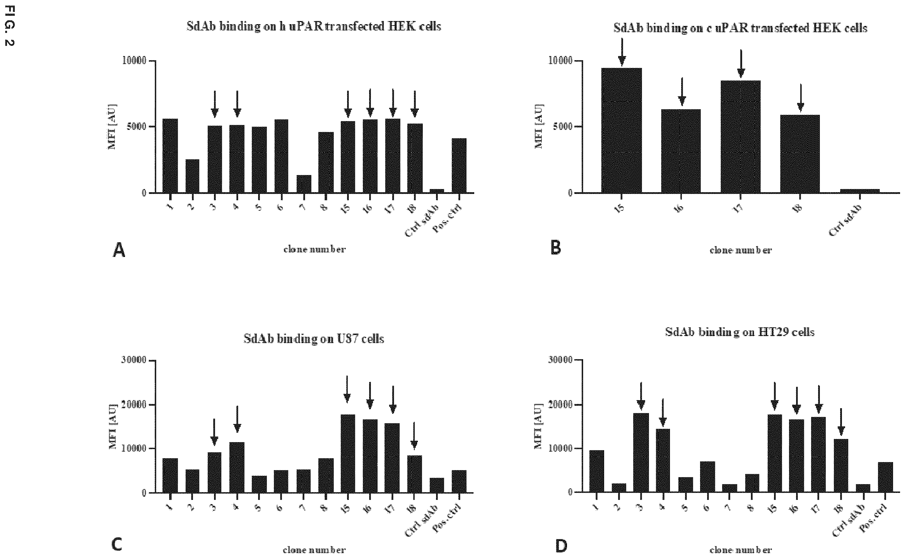

**Figure 2** shows a graphical representation of the mean fluorescent intensity (MFI) values obtained during flow cytometry screening of some of the selected sdAbs (as described in example 1). SdAbs selected for further *in vivo* evaluation are indicated with arrows, including sdAb 15, an immunoglobulin single variable domain according to an embodiment of the present invention.

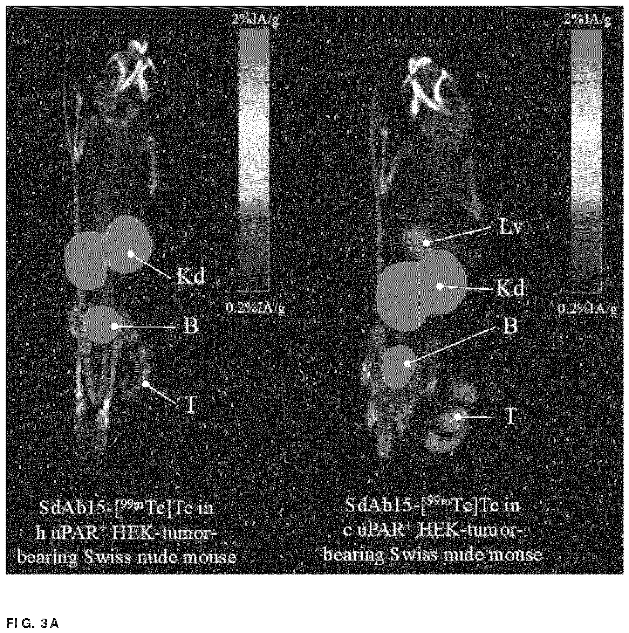

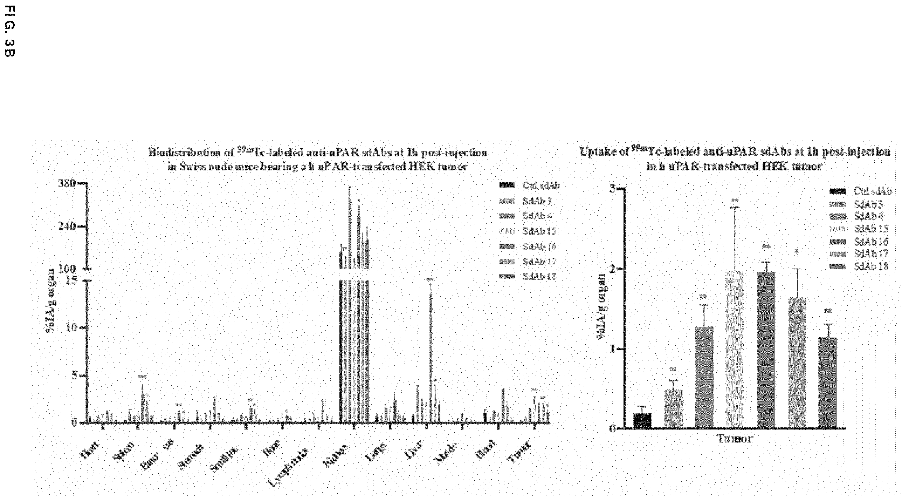

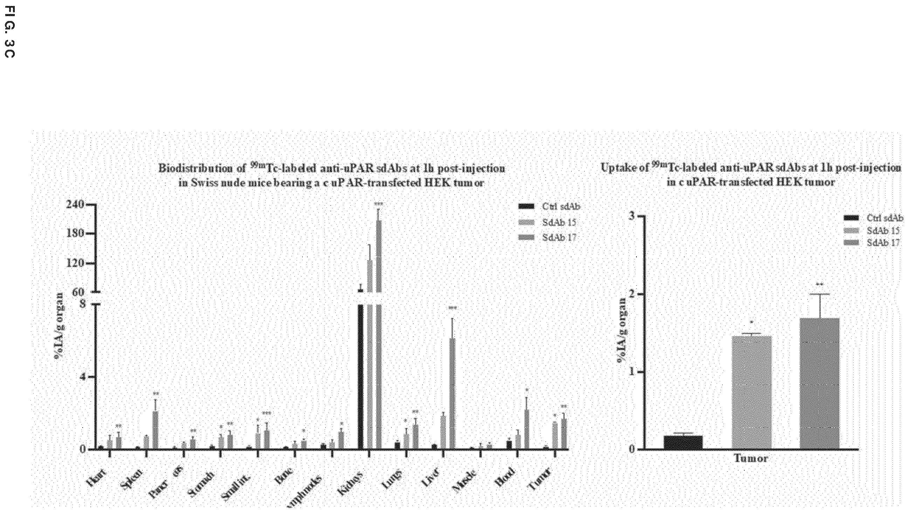

**Figure 3A** shows biodistribution of ^{99m}Tc-labeled anti-uPAR sdAb 15, an immunoglobulin single variable domain according to an embodiment of the present invention in Swiss nude mice bearing uPAR-transduced HEK tumors. Micro-SPECT/CT images were obtained 1 h after intravenous injection of ^{99m}Tc-labeled sdAbs. Arrows indicate T: tumor; B: bladder; Kd: kidneys; Lv: liver and Lu: lungs. **Figures 3B -3C** shows a graphical representation of the biodistribution and tumor targeting of the ^{99m}Tc-labeled anti-uPAR sdAbs (including sdAb 15, an immunoglobulin single variable domain according to an embodiment of the present invention) and non-targeting control sdAb in Swiss nude mice bearing transduced HEK tumors. Statistical analyses were performed using a Kruskal-Wallis test followed by a Dunn's multiple comparisons test for the selection of the lead sdAbs. Statistical significance was set at p<0.05 (^{∗}p<0.05, ^{∗∗}p<0.01, ^{∗∗∗}p<0.001, ^{∗∗∗∗}p<0.001).

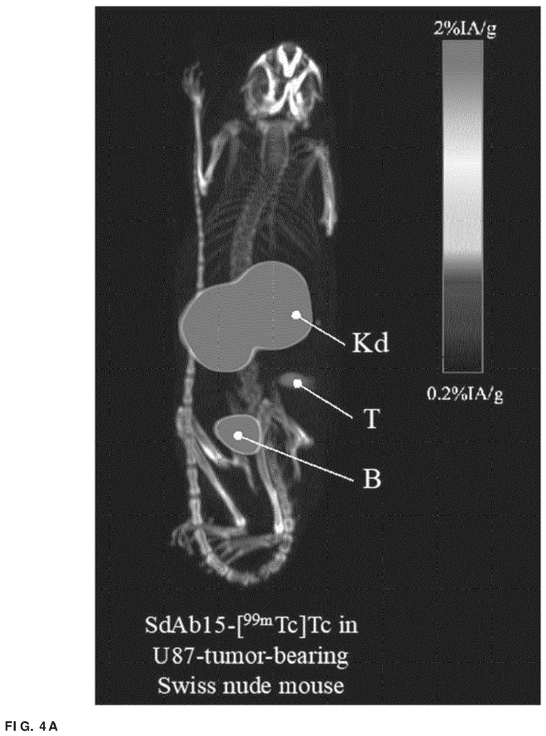

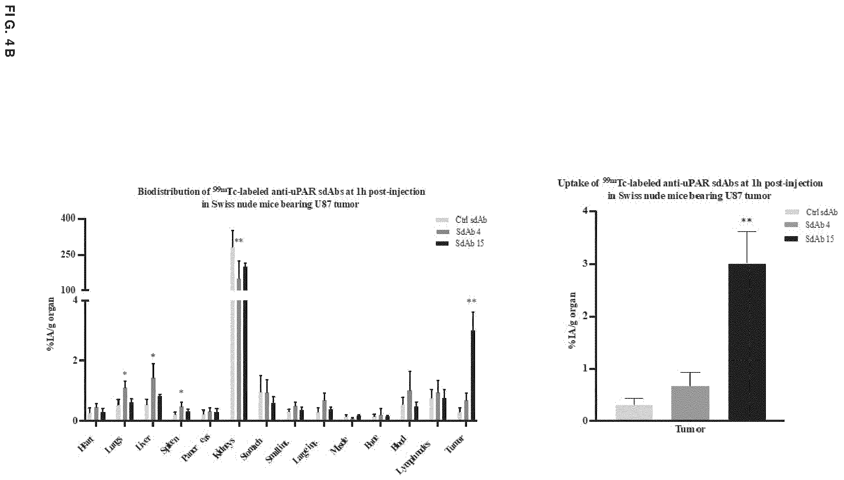

**Figure 4A** shows biodistribution of ^{99m}Tc-labeled sdAb 15 (an immunoglobulin single variable domain according to an embodiment of the present invention) in Swiss nude bearing a subcutaneous U87 tumor. Micro-SPECT/CT images were obtained 1 h after intravenous injection of ^{99m}Tc-labeled sdAbs. Arrows indicate Kd: kidneys; T: tumor; B: bladder; Lu: lungs and Lv: liver. **Figure 4B** shows a graphical representation of the biodistribution and tumor targeting of anti-human uPAR sdAbs-[^{99m}Tc]Tc (including sdAb 15, an immunoglobulin single variable domain according to an embodiment of the present invention) together with radiolabeled non-targeting control sdAb in Swiss nude bearing a subcutaneous U87 tumor. Statistical analyses were performed using a Kruskal-Wallis test followed by a Dunn's multiple comparisons test for the selection of the lead sdAbs. For all tests statistical significance was set at p<0.05 (^{∗}p<0.05, ^{∗∗}p<0.01, ^{∗∗∗}p<0.001, ^{∗∗∗∗}p<0.001).

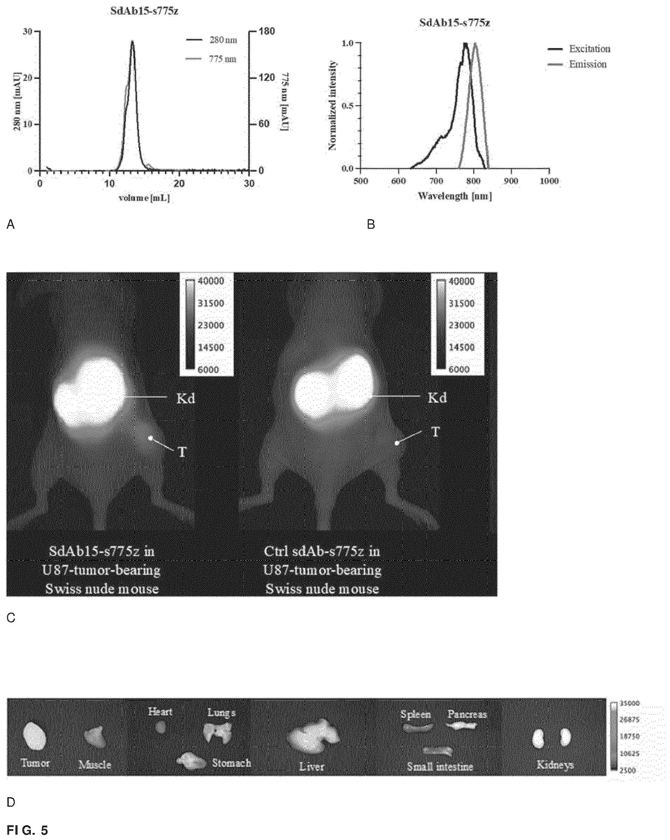

**Figure 5A** shows a chromatogram and **Figure 5B** shows excitation and emission spectra of sdAb 15 (an immunoglobulin single variable domain according to an embodiment of the present invention) randomly labeled with s775z, representing a desired profile for randomly labeled sdAb. **Figure 5C** shows *in vivo* fluorescence imaging of sdAb15-s775z with corresponding non-targeting (control) R3B23-s775z in mice bearing a subcutaneous U87 tumor. Fluorescence images were obtained 1 h after intravenous injection of labeled sdAbs. Arrows indicate Kd: kidneys and T: tumor. **Figure 5D** shows *ex vivo* fluorescence imaging of organ biodistribution of s775z-sdAb15.

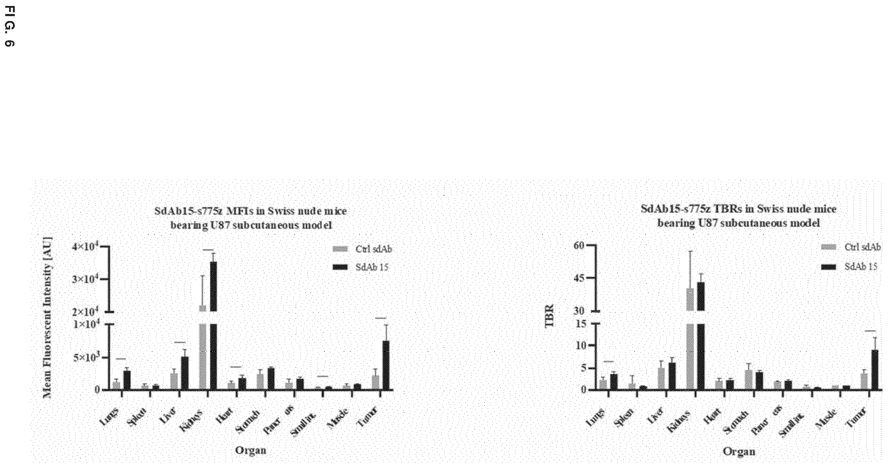

**Figure 6** shows a graphical representation of MFI and Tumor to Background Ration (TBR) of dissected organs of sdAb15-s775z (a conjugate according to an embodiment of the present invention), grouped together with values obtained from corresponding control. Statistical analyses were performed using a Mann-Whitney test for comparison of MFI and TBR values between targeting and control sdAb. Statistical significance was set at p<0.05 (^{∗}p<0.05).

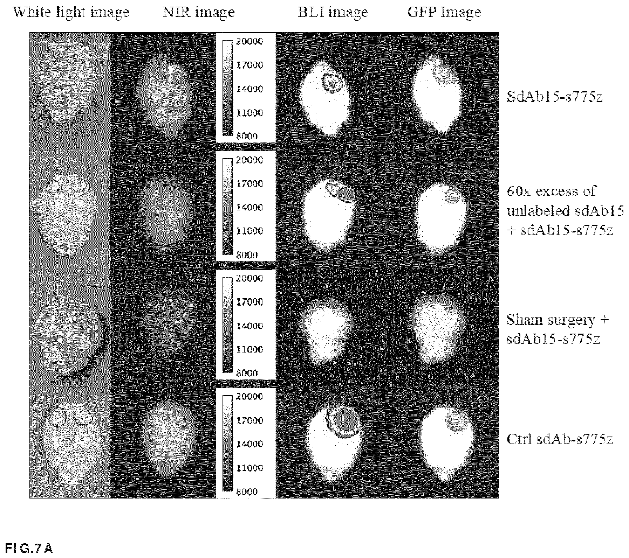

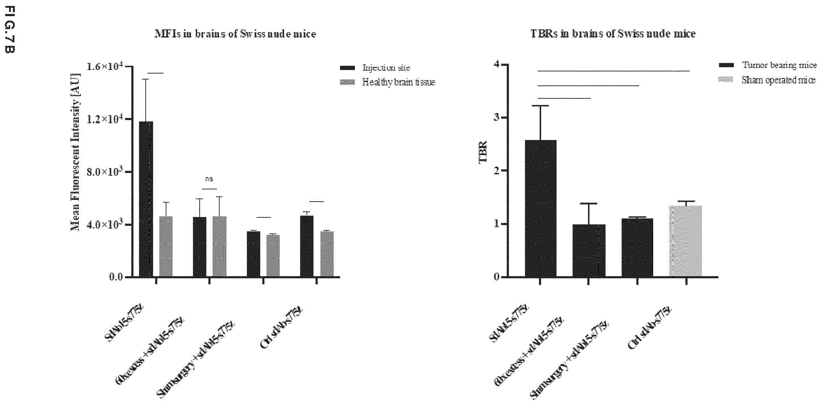

**Figure 7** shows (A) *ex vivo* brain imaging 1 h after intravenous injection of fluorescently-labeled sdAbs (including sdAb15-s775z, a conjugate according to an embodiment of the present invention) in U87 orthotopic tumor bearing or sham operated mice. Regions of interest (ROIs) were drawn based on the GFP image (or injection site for sham surgery) and are indicated on white light image (injection sites and healthy tissues are indicated on right and left hemispheres, respectively). (B) Graphical representation of MFIs of tumors and healthy brain tissue and quantified TBRs. Statistical analyses were performed using a Mann-Whitney test. Healthy brain tissue was used as a control for MFI. TBR values were compared between targeting sdAb and control conditions. Statistical significance was set at p<0.05 (^{∗}p<0.05).

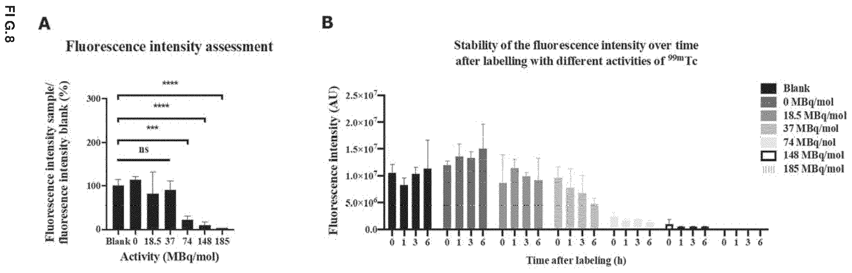

**Figure 8** shows (A) The fluorescence intensity of bimodal-labeled anti-uPAR sdAb 15 (a conjugate according to an embodiment of the present invention) with increased MBq/mol ^{99m}Tc. Fluorescent signal is well preserved until 37 MBq/mol ^{99m}Tc. Results are presented as mean ± st. dev. of the % intensity to the standard. (B) The stability of the fluorescence intensity of bimodal sdAb 15 at 0, 1, 3, and 6 h after radiolabeling. Results are presented as mean ± st. dev. of the absolute fluorescent intensity.

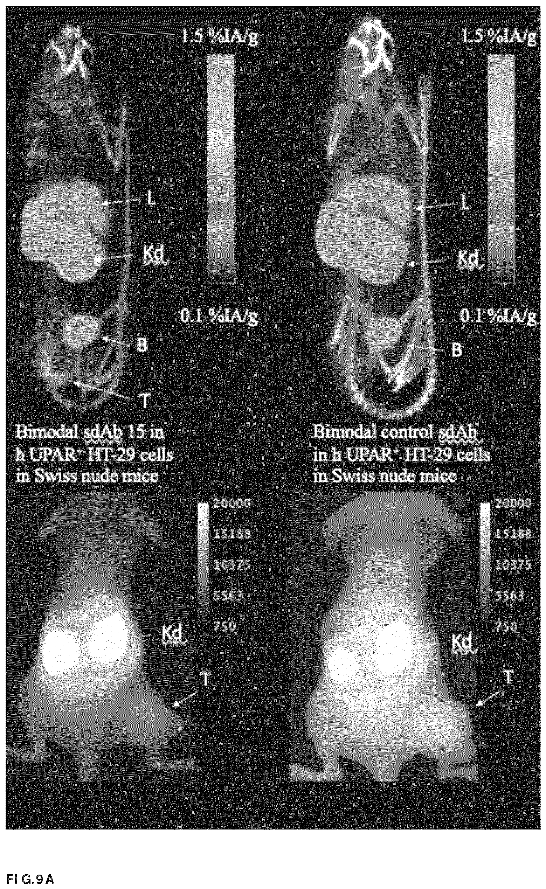

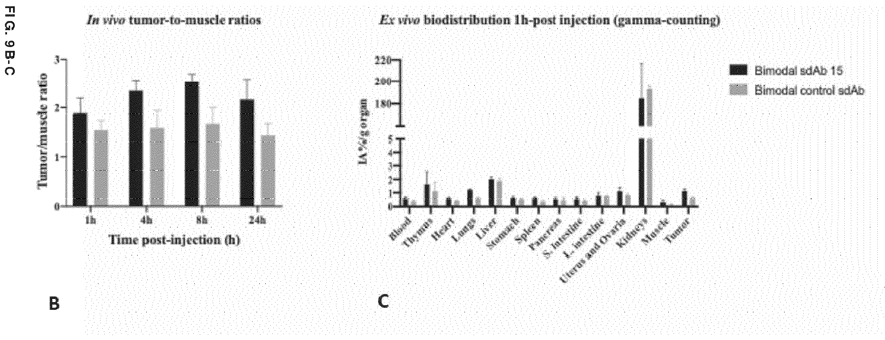

**Figure 9** shows (A) Biodistribution of bimodal-labeled anti-uPAR sdAb 15 (a conjugate according to an embodiment of the present invention) and control sdAb, in Swiss nude mice bearing h uPAR⁺ HT-29 tumors. Micro-SPECT/CT imaging and fluorescence imaging obtained at 4 h after intravenous injection of the bimodal sdAbs. Arrows indicate T: tumor; B: bladder; Kd: kidneys and L: liver. (B) Graphical representation of the *in vivo* TBRs based on fluorescence imaging of the bimodal-labeled anti-uPAR sdAb 15 and non-targeting control sdAb in Swiss nude mice bearing h uPAR⁺ tumors. (C) Graphical representation of the *ex vivo* biodistribution and tumor targeting of the bimodal anti-uPAR sdAb 15 and the non-targeting control sdAb based on *ex vivo* organ analysis of h uPAR⁺ tumor bearing Swiss nude mice. Statistical analyses were performed using a Kruskal-Wallis test followed by a Dunn's multiple comparisons test.

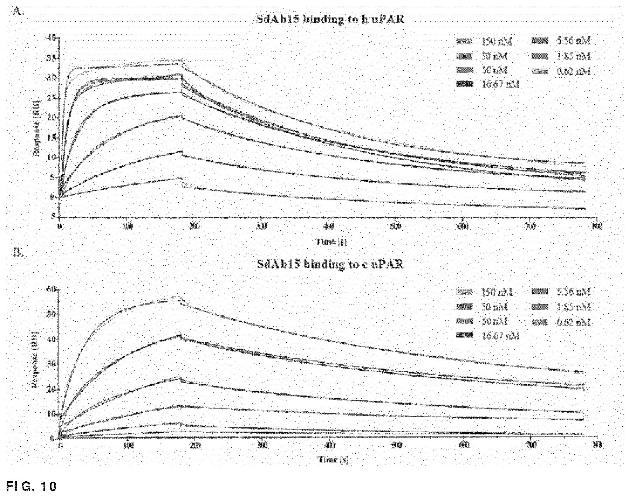

**Figure 10** shows sensorgrams of different concentrations (as indicated in the graph) of sdAb15 (an immunoglobulin single variable domain according to an embodiment of the present invention) binding to recombinant h (A) and c (B) uPAR. Kinetics were measured with a three-fold dilution series of the sdAbs (150-0.62 nM). The fitting of the binding curves using the '1:1 binding with drift and RI2' model was applied to calculate kinetic parameters.

### DETAI LED DESCRIPTION OF THE INVENTION

The present invention concerns immunoglobulin single variable domains directed against human urokinase plasminogen activator receptor (uPAR). More specifically, it concerns immunoglobulin single variable domains against human uPAR, showing cross-reactivity against canine uPAR, and their use in targeting and *in vivo* imaging of tumor and tumor-associated cells, with applications in the field of cancer diagnostics and therapeutics and monitoring of the disease.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

"Immunoglobulin single variable domain" as used herein defines molecules wherein the antigen binding site is present on, and formed by, a single immunoglobulin domain (which is different from conventional immunoglobulins or their fragments, wherein typically two immunoglobulin variable domains interact to form an antigen binding site). It should however be clear that the term "immunoglobulin single variable domain" does comprise fragments of conventional immunoglobulins wherein the antigen binding site is formed by a single variable domain.

Generally, an immunoglobulin single variable domain will have an amino acid sequence comprising 4 framework regions (FR1 to FR4) and 3 complementarity determining regions (CDR1 to CDR3), preferably according to the following formula (1): FR1-CDR1-FR2-CDR2-FR3-CDR3-FR4 (1), or any suitable fragment thereof (which will then usually contain at least some of the amino acid residues that form at least one of the complementarity determining regions). Immunoglobulin single variable domains comprising 4 FRs and 3 CDRs are known to the person skilled in the art and have been described.

Typical, but non-limiting, examples of immunoglobulin single variable domains include light chain variable domain sequences (e.g. a VL domain sequence) or a suitable fragment thereof, or heavy chain variable domain sequences (e.g. a VH domain sequence or V_{H}H domain sequence) or a suitable fragment thereof, as long as it is capable of forming a single antigen binding unit. Thus, according to a preferred embodiment, the immunoglobulin single variable domain is a light chain variable domain sequence (e.g. a VL domain sequence) or a heavy chain variable domain sequence (e.g. a VH domain sequence); more specifically, the immunoglobulin single variable domain is a heavy chain variable domain sequence that is derived from a conventional four-chain antibody or a heavy chain variable domain sequence that is derived from a heavy chain antibody. The immunoglobulin single variable domain may be a domain antibody ("dAB" or "dAb"), or a single domain antibody ("sdAB" or "sdAb"), or a V_{H}H domain sequence or another immunoglobulin single variable domain, or any suitable fragment of any one thereof. The immunoglobulin single variable domains, generally comprise a single amino acid chain that can be considered to comprise 4 "framework sequences" or FR's and 3 "complementary determining regions" or CDR's (as defined herein). It should be clear that framework regions of immunoglobulin single variable domains may also contribute to the binding of their antigens. The delineation of the CDR sequences (and thus also the FR sequences) can be based on the IMGT unique numbering system for V-domains and V-like domains. Alternatively, the delineation of the FR and CDR sequences can be done by using the Kabat numbering system as applied to V_{H}H domains from Camelids.

It should be noted that the immunoglobulin single variable domains as binding domain moiety in their broadest sense are not limited to a specific biological source or to a specific method of preparation. The term "immunoglobulin single variable domain" encompasses variable domains of different origin, comprising mouse, rat, rabbit, donkey, human, shark or camelid variable domains. According to specific embodiments, the immunoglobulin single variable domains are derived from shark antibodies (the so-called immunoglobulin new antigen receptors or IgNARs), more specifically from naturally occurring heavy chain shark antibodies, devoid of light chains, and are known as VNAR domain sequences. Preferably, the immunoglobulin single variable domains are derived from camelid antibodies. More preferably, the immunoglobulin single variable domains are derived from naturally occurring heavy chain camelid antibodies, devoid of light chains, and are known as V_{H}H domain sequences.

The term "V_{H}H domain sequence" is, as used herein, is interchangeably with the term "single domain antibody fragment (sdAb)" and refers to a single domain antigen binding fragment. It particularly refers to a single variable domain derived from naturally occurring heavy chain antibodies and is known to the person skilled in the art. V_{H}H domain sequences are usually derived from heavy chain only antibodies (devoid of light chains) seen in camelids and consequently are often referred to as V_{H}H antibody or V_{H}H sequence. Camelids comprise old world camelids (Camelus bactrianus and Camelus dromedarius) and new world camelids (for example Lama pacos, Lama glama, Lama guanicoe, Vicugna pacos and Lama vicugna). The small size and unique biophysical properties of V_{H}H domain sequences excel conventional antibody fragments for the recognition of uncommon or hidden epitopes and for binding into cavities or active sites of protein targets. V_{H}H domain sequences are stable, survive the gastro-intestinal system and can easily be manufactured. Therefore, V_{H}H domain sequences can be used in many applications including drug discovery and therapy, but also as a versatile and valuable tool for purification, functional study and crystallization of proteins.

The V_{H}H domain sequences of the invention generally comprise a single amino acid chain that can be considered to comprise 4 "framework regions" or FR's and 3 "complementarity determining regions" or CDR's, according to formula (1) (as defined above). The term "complementarity determining region" or "CDR" refers to variable regions in V_{H}H domain sequences and contains the amino acid sequences capable of specifically binding to antigenic targets. These CDR regions account for the basic specificity of the V_{H}H domain sequences for a particular antigenic determinant structure. Such regions are also referred to as "hypervariable regions." The V_{H}H domain sequences have 3 CDR regions, each non-contiguous with the others (termed CDR1, CDR2, CDR3). The delineation of the FR and CDR sequences is often based on the IMGT unique numbering system for V-domains and V-like domains. Alternatively, the delineation of the FR and CDR sequences can be done by using the Kabat numbering system as applied to V_{H}H domains from Camelids. As will be known by the person skilled in the art, the V_{H}H domain sequences can in particular be characterized by the presence of one or more Camelidae hallmark residues in one or more of the framework sequences (according to Kabat numbering).

One preferred class of immunoglobulin single variable domains of the current invention corresponds to the V_{H}H domains of naturally occurring heavy chain antibodies, also called "V_{H}H domain sequences" or "single domain antibody fragment (sdAb)".

Such V_{H}H domain sequences can generally be generated or obtained by suitably immunizing a species of Camelid with a desired target, (i.e. so as to raise an immune response and/or heavy chain antibodies directed against a desired target), by obtaining a suitable biological sample from said Camelid (such as a blood sample, or any sample of B-cells), and by generating V_{H}H sequences directed against the desired target, starting from said sample, using any suitable technique known per se. Such techniques will be clear to the skilled person. Alternatively, such naturally occurring V_{H}H domains against the desired target can be obtained from naive libraries of Camelid V_{H}H sequences, for example by screening such a library using the desired target or at least one part, fragment, antigenic determinant or epitope thereof using one or more screening techniques known per se. Such libraries and techniques are for example described in 9937681, WO0190190, WO03025020 and WO03035694. Alternatively, improved synthetic or semi-synthetic libraries derived from naive V_{H}H libraries may be used, such as V_{H}H libraries obtained from naive V_{H}H libraries by techniques such as random mutagenesis and/or CDR shuffling, as for example described in WO0043507. Yet another technique for obtaining V_{H}H domain sequences directed against a desired target involves suitably immunizing a transgenic mammal that is capable of expressing heavy chain antibodies (i.e. so as to raise an immune response and/or heavy chain antibodies directed against a desired target), obtaining a suitable biological sample from said transgenic mammal (such as a blood sample, or any sample of B-cells), and then generating V_{H}H domain sequences directed against the desired target starting from said sample, using any suitable technique known per se. For example, for this purpose, the heavy chain antibody-expressing mice and the further methods and techniques described in WO02085945 and in WO04049794 can be used.

A particularly preferred class of immunoglobulin single variable domains of the invention comprises V_{H}H domain sequences with an amino acid sequence that corresponds to the amino acid sequence of a naturally occurring V_{H}H domain, but that has been "humanized" , i.e. by replacing one or more amino acid residues in the amino acid sequence of said naturally occurring V_{H}H sequence (and in particular in the framework sequences) by one or more of the amino acid residues that occur at the corresponding position(s) in a VH domain from a conventional 4-chain antibody from a human being. This can be performed in a manner known per se, which will be clear to the skilled person and on the basis of the prior art on humanization. Again, it should be noted that such humanized V_{H}H domain sequences of the invention can be obtained in any suitable manner known per se and thus are not strictly limited to polypeptides that have been obtained using a polypeptide that comprises a naturally occurring V_{H}H domain as a starting material. Humanized V_{H}H domain sequences may have several advantages, such as a reduced immunogenicity, compared to the corresponding naturally occurring V_{H}H domains. Such humanization generally involves replacing one or more amino acid residues in the sequence of a naturally occurring V_{H}H with the amino acid residues that occur at the same position in a human VH domain, such as a human VH3 domain. The humanizing substitutions should be chosen such that the resulting humanized V_{H}H domain sequences still retain the favourable properties of V_{H}H domain sequences as defined herein. The skilled person will be able to select humanizing substitutions or suitable combinations of humanizing substitutions which optimize or achieve a desired or suitable balance between the favourable properties provided by the humanizing substitutions on the one hand and the favourable properties of naturally occurring V_{H}H domains on the other hand.

Also within the scope of the invention are natural or synthetic analogs, mutants, variants, alleles, homologs and orthologs of the immunoglobulin single variable domain of the invention as defined herein.

By means of non-limiting examples, a substitution may for example be a conservative substitution (as described herein) and/or an amino acid residue may be replaced by another amino acid residue. Thus, any one or more substitutions, deletions or insertions, or any combination thereof, that either improve the properties of the immunoglobulin single variable domain of the invention or that at least do not detract too much from the desired properties or from the balance or combination of desired properties of the immunoglobulin single variable domain of the invention (i.e. to the extent that the immunoglobulin single variable domain is no longer suited for its intended use) are included within the scope of the invention. A skilled person will generally be able to determine and select suitable substitutions, deletions or insertions, or suitable combinations of thereof, based on the disclosure herein and optionally after a limited degree of routine experimentation, which may for example involve introducing a limited number of possible substitutions and determining their influence on the properties of the immunoglobulin single variable domain thus obtained.

Further, depending on the host organism used to express the immunoglobulin single variable domain of the invention, such deletions and/or substitutions may be designed in such a way that one or more sites for post-translational modification (such as one or more glycosylation sites) are removed, as will be within the ability of the person skilled in the art. Alternatively, substitutions or insertions may be designed so as to introduce one or more sites for attachment of functional groups.

Examples of modifications, as well as examples of amino acid residues within the immunoglobulin single variable domain sequence, that can be modified, methods and techniques that can be used to introduce such modifications and the potential uses and advantages of such modifications will be clear to the skilled person. For example, such a modification may involve the introduction (e.g. by covalent linking or in another suitable manner) of one or more functional groups, residues or moieties into or onto the immunoglobulin single variable domain, and in particular of one or more functional groups, residues or moieties that confer one or more desired properties or functionalities to the immunoglobulin single variable domain of the invention. Examples of such functional groups and of techniques for introducing them will be clear to the skilled person, and can generally comprise all functional groups and techniques mentioned in the general background art cited hereinabove as well as the functional groups and techniques known per se for the modification of pharmaceutical proteins, and in particular for the modification of antibodies or antibody fragments (including single domain antibody fragments). Such functional groups may for example be linked directly (for example covalently) to an immunoglobulin single variable domain of the invention, or optionally via a suitable linker or spacer, as will again be clear to the skilled person. One of the most widely used techniques for increasing the half-life and/or reducing immunogenicity of pharmaceutical proteins comprises attachment of a suitable pharmacologically acceptable polymer, such as poly(ethyleneglycol) (PEG) or derivatives thereof (such as methoxypoly(ethyleneglycol) or mPEG). Generally, any suitable form of pegylation can be used, such as the pegylation used in the art for antibodies and antibody fragments. In an embodiment, site-directed pegylation is used, in particular via a cysteine-residue. For example, for this purpose, PEG may be attached to a cysteine residue that naturally occurs in an immunoglobulin single variable domain of the invention, an immunoglobulin single variable domain of the invention may be modified so as to suitably introduce one or more cysteine residues for attachment of PEG, or an amino acid sequence comprising one or more cysteine residues for attachment of PEG may be fused to the N- and/or C-terminus of an immunoglobulin single variable domain of the invention, all using techniques of protein engineering known per se to the skilled person. Another, usually less preferred modification comprises N-linked or O-linked glycosylation, usually as part of co-translational and/or post-translational modification, depending on the host cell used for expressing immunoglobulin single variable domain of the invention.

As used herein, the terms "nucleic acid molecule", "polynucleotide", "polynucleic acid", "nucleic acid" are used interchangeably and refer to a polymeric form of nucleotides of any length, either deoxyribonucleotides or ribonucleotides, or analogs thereof. Polynucleotides may have any three- dimensional structure, and may perform any function, known or unknown. Non-limiting examples of polynucleotides include a gene, a gene fragment, exons, introns, messenger RNA (mRNA), transfer RNA, ribosomal RNA, ribozymes, cDNA, recombinant polynucleotides, branched polynucleotides, plasmids, vectors, isolated DNA of any sequence, control regions, isolated RNA of any sequence, nucleic acid probes, and primers. The nucleic acid molecule may be linear or circular.

The term "sequence identity" as used herein refers to the extent that sequences are identical on a nucleotide-by-nucleotide basis or an amino acid-by-amino acid basis over a window of comparison. Thus, a "percentage of sequence identity" is calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, lie, Phe, Tyr, Trp, Lys, Arg, His, Asp, Glu, Asn, Gin, Cys and Met) occurs in both sequences to yield the number of matched positions, dividing the number of matched positions by the total number of positions in the window of comparison (i.e., the window size), and multiplying the result by 100 to yield the percentage of sequence identity. Determining the percentage of sequence identity can be done manually, or by making use of computer programs that are available in the art. Examples of useful algorithms are PILEUP (Higgins & Sharp, CABIOS 5:151 (1989), BLAST and BLAST 2.0 (Altschul et al. J. Mol. Biol. 215: 403 (1990). Software for performing BLAST analyses is publicly available through the National Center for Biotechnology Information (http://www.ncbi.nlm.nih.gov/). A "deletion" is defined here as a change in either amino acid or nucleotide sequence in which one or more amino acid or nucleotide residues, respectively, are absent as compared to an amino acid sequence or nucleotide sequence of a parental polypeptide or nucleic acid. Within the context of a protein, a deletion can involve deletion of about 2, about 5, about 10, up to about 20, up to about 30 or up to about 50 or more amino acids. A protein or a fragment thereof may contain more than one deletion.

An "insertion" or "addition" is that change in an amino acid or nucleotide sequences which has resulted in the addition of one or more amino acid or nucleotide residues, respectively, as compared to an amino acid sequence or nucleotide sequence of a parental protein. "Insertion" generally refers to addition to one or more amino acid residues within an amino acid sequence of a polypeptide, while "addition" can be an insertion or refer to amino acid residues added at an N- or C-terminus, or both termini. Within the context of a protein or a fragment thereof, an insertion or addition is usually of about 1, about 3, about 5, about 10, up to about 20, up to about 30 or up to about 50 or more amino acids. A protein or fragment thereof may contain more than one insertion. A "substitution", as used herein, results from the replacement of one or more amino acids or nucleotides by different amino acids or nucleotides, respectively as compared to an amino acid sequence or nucleotide sequence of a parental protein or a fragment thereof. It is understood that a protein or a fragment thereof may have conservative amino acid substitutions which have substantially no effect on the protein's activity. By conservative substitutions is intended combinations such as gly, ala; val, ile, leu, met; asp, glu; asn, gin; ser, thr; lys, arg; cys, met; and phe, tyr, trp.

As used herein, the term "cancer" or "tumor" refers to any neoplastic disorder, including prostate cancer, liver cancer, head and/or neck cancer (e.g. throat cancer, pharyngeal squamous cell carcinoma), brain cancer, skin cancer (e.g. melanoma), bladder cancer, ovarian cancer (e.g. ovarian carcinoma), uterus cancer, lung cancer, breast cancer (e.g. mammary adenocarcinoma), sarcoma, cervix cancer, vulva cancer, hematologic cancers (e.g. chronic leukemia, mastocytoma), renal cancer (e.g. renal cell cancer), bone cancer, and/or gastrointestinal cancers (e.g. colorectal cancer, pancreas cancer, gastric cancer, esophageal cancer).

"Radionuclide" as used herein refers to an atom that has excess nuclear energy, making it unstable. This excess energy can be used in one of three ways: emitted from the nucleus as gamma radiation; transferred to one of its electrons to release it as a conversion electron; or used to create and emit a new particle (alpha particle or beta particle) from the nucleus.

As used herein, the term "diagnosing" or grammatically equivalent wordings, means determining whether or not a subject suffers from a particular disease or disorder.

As used herein, "prognosing" or grammatically equivalent wordings, means determining whether or not a subject has a risk of developing a particular disease or disorder.

As used herein, "treating cancer" or "treating a subject or individual having cancer" or "cancer treatment" includes substantially inhibiting the disease, substantially slowing or reversing the progression of the disease, substantially ameliorating clinical symptoms of the disease or substantially preventing the appearance of clinical symptoms of the disease. In particular, it includes inhibition of the replication of cancer cells, inhibition of the spread of cancer, reduction in tumor size, lessening or reducing the number of cancerous cells in the body, and/or amelioration or alleviation of the symptoms of cancer. A treatment is considered therapeutic if there is a decrease in mortality and/or morbidity, and may be performed prophylactically, or therapeutically.

Generally the "subjects" are mammals or mammalian, where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In many embodiments, the subjects will be humans.

As used herein, "image-guided therapy" refers to the use of any form of medical imaging to plan, perform, and evaluate surgical procedures and therapeutic interventions.

"Bimodal label" as used herein refers to a detectable label comprising a fluorescent moiety and a radionuclide.

"Targeting moiety" or "targeting agent" as used herein refers to an agent possessing binding affinity for a specific molecular target or biomarker. In the current invention such "targeting moiety" or "targeting agent" is coupled to a detectable label to form a targeted tracer that can for instance be used for *in vivo* medical imaging or is coupled to a functional moiety that can for instance be used in cancer therapy.

### Description

Urokinase plasminogen activator receptor (uPAR) is part of the plasminogen activator (PA) system, which has been shown to be of special importance during cancer invasion and metastasis. The PA system consists of the serine protease urokinase-type plasminogen (uPA), its cell membrane receptor (uPAR), the substrate plasminogen and the plasminogen activator inhibitors PAI-1 and PAI-2. The urokinase(-type) plasminogen activator receptor (uPAR or CD87 (Cluster of Differentiation 87)) is a glycosylated protein of 45-55 kDa consisting of three homologous cysteine-rich domains (D1, D2, D3). The protein is localized to the extracellular leaf of the plasma membrane through a glycosylphosphatidylinositol (GPI) anchor. UPAR mediates a wide variety of cellular processes including inflammation, metastasis and invasion, tissue remodeling, angiogenesis, and cell adhesion.

Many of these processes are initiated by the highly specific binding of various ligands to membrane-bound uPAR at the cell surface. One such interaction is between uPAR and uPA, which mediates both extracellular and intracellular signaling events. Binding of extracellular pro-uPA to uPAR facilitates its activation. In turn, uPA activates proteases, such as plasmin, which directly and indirectly degrade the extracellular matrix (ECM). Furthermore, plasmin can activate pro-uPA leading to a positive feedback loop that accelerates ECM degradation. uPAR is also able to act intracellularly by activating proliferative signal transduction pathways. uPAR is believed to directly associate with integrin family adhesion receptors in complexes that mediate RGD-independent cell signaling and migration. Accordingly, uPAR plays a role in the development of cancer and the metastasis of cancer.

Due to the importance of uPAR in cancer invasion and metastasis, a number of uPAR-targeting molecules have been identified during the last decades. Conjugates of targeting molecules and detectable labels can be used as tracers for *in vivo* medical imaging. For instance, PET imaging of uPAR overexpression has been proposed for the prediction of poor prognosis and cancer aggressiveness. Moreover, the paramount presence of uPAR on the invasive front of the tumor makes this receptor especially attractive for intraoperative fluorescence imaging. Furthermore, said targeting molecules can also be used in molecular therapy for the targeted delivery of toxic substances such as drugs or therapeutic radiation. The specific accumulation of a detectable label or therapeutic compound in the tissue of interest, requires the stable conjugation of said detectable label or therapeutic compound to a targeting molecule such as an antibody, antibody-fragment, protein scaffold, peptide or small molecule, recognizing a specific biomarker.

Examples of such human uPAR-targeting peptides include a dimeric linear peptide (AE120) or a monomer version thereof (AE105). However, studies with these targeting peptides show a suboptimal *in vivo* biodistribution profile.

Likewise, full-sized monoclonal antibodies (MAbs) have a number of disadvantages that have so far limited their effective use as targeting agents for tracers in the clinic. MAbs are macromolecules with a relatively poor penetration into solid and isolated tissues such as tumors. In addition, complete MAbs feature a long residence time in the body and a potential increase in background signals because of binding to Fcreceptors on non-target cells, making them less suitable for molecular imaging applications. Indeed, for imaging the most important properties of a tracer are: rapid interaction with the target, fast clearing of unbound molecules from the body and low non-specific accumulation, especially around the area of interest. These requirements have led to the development of a myriad of antibody derived probe formats, trying to combine specificity with a small size for favorable pharmacokinetics.

Immunoglobulin single variable domains are such antibody derived molecules wherein the antigen binding site is present on, and formed by, a single immunoglobulin domain (which is different from conventional immunoglobulins or their fragments, wherein typically two immunoglobulin variable domains interact to form an antigen binding site). Immunoglobulin single variable domains exhibit faster pharmacokinetics as compared to monoclonal antibodies or larger antibodyfragments. This results in higher contrast and shorter waiting times after intravenous injection.

In a first aspect, the invention relates to an immunoglobulin single variable domain that is directed against and/or that specifically binds to human urokinase plasminogen activator receptor (human uPAR, SEQ ID NO: 1), wherein the immunoglobulin single variable domain comprises polypeptides that have at least 90%, preferably at least 91%, preferably at least 92%, preferably at least 93%, preferably at least 94%, preferably at least 95%, preferably at least 96%, preferably at least 97%, preferably at least 98%, preferably at least 99%, preferably at least 100% amino acid sequence identity with SEQ ID NO: 2. In an embodiment, the immunoglobulin single variable domain comprises polypeptides that have 1, 2 or 3 amino acid difference with SEQ ID NO: 2. In an embodiment, the immunoglobulin single variable domain comprises an amino acid sequence according to SEQ ID NO: 2.

In an embodiment, the immunoglobulin single variable domain has at least 90%, preferably at least 91%, preferably at least 92%, preferably at least 93%, preferably at least 94%, preferably at least 95%, preferably at least 96%, preferably at least 97%, preferably at least 98%, preferably at least 99%, preferably at least 100% amino acid sequence identity with SEQ ID NO: 2. In an embodiment, the immunoglobulin single variable domain has 1, 2 or 3 amino acid difference with SEQ ID NO: 2. In an embodiment, the immunoglobulin single variable domain comprises an amino acid sequence according to SEQ ID NO: 2.

As such, the invention relates to an immunoglobulin single variable domain that is directed against and/or that specifically binds to human urokinase-plasminogen activator receptor (human uPAR, SEQ ID NO: 1), wherein the immunoglobulin single variable domain comprises an amino acid sequence chosen from the group consisting of:
a. SEQ ID NO: 2
b. Polypeptides that have at least 90% amino acid identity with SEQ ID NO: 2,
c. Polypeptides that have 1, 2 or 3 amino acid difference with SEQ ID NO: 2.

The inventors found that the immunoglobulin single variable domains of the invention possess an especially favourable *in vivo* biodistribution profile, including rapid pharmacokinetics, with rapid blood clearance and elimination through the kidneys, fast and homogenous tumor uptake, and low non-specific uptake in organs with no uPAR expression.

The term "urokinase plasminogen activator receptor" (uPAR), as used herein, is known in the art and refers to a glycosylated protein of 45-55 kDa consisting of three homologous cysteine-rich domains. The protein is localized to the extracellular leaf of the plasma membrane through a glycosylphosphatidylinositol anchor. UPAR mediates a wide variety of cellular processes including inflammation, metastasis and invasion, tissue remodeling, angiogenesis, and cell adhesion. In particular, the human urokinase plasminogen activator receptor is known as CD87 (Cluster of Differentiation 87) or PLAUR (plasminogen activator, urokinase receptor) (accession number nucleotide sequence: NM_002659; accession number protein sequence: NP_002650.1 and as in SEQ ID NO: 1).

The present invention is in its broadest sense not particularly limited to or defined by a specific antigenic determinant, epitope, part, domain, subunit or conformation of human uPAR (SEQ ID NO: 1) against which the immunoglobulin single variable domains are directed. It is expected that the immunoglobulin single variable domains according to the invention will generally bind to all naturally occurring or synthetic analogs, variants, mutants, alleles of the uPARs mentioned herein. The human urokinase plasminogen activator receptor as referred to in the present invention also includes fragments of the full-length human uPAR protein. A non-limiting example of a fragment of the full-length uPAR protein includes the extracellular domain of a particular uPAR. The extracellular domain of the human urokinase plasminogen activator receptor is defined as the AA 23-AA 305 (LEU23 - GLY305) fragment (SEQ ID NO: 23) of the corresponding full-length human uPAR amino acid sequence as defined in NP_002650.1 (SEQ ID NO: 1). Thus, according to a preferred embodiment, the immunoglobulin single variable domain specifically binds to the extracellular domain of the human urokinase plasminogen activator receptor (SEQ ID NO: 23).

As used herein, the term "specifically recognizing" or "specifically binding to" or simply "specific for" refers to the ability of an immunoglobulin single variable domain to preferentially bind to a particular antigen that is present in a homogeneous mixture of different antigens and does not necessarily imply high affinity (as defined further herein). In certain embodiments, a specific binding interaction will discriminate between desirable and undesirable antigens in a sample, in some embodiments more than about 10 to 100-fold or more (e.g., more than about 1000or 10,000-fold). The terms "specifically bind", "selectively bind", "preferentially bind", and grammatical equivalents thereof, are used interchangeably herein. The term "affinity", as used herein, refers to the degree to which an immunoglobulin single variable domain binds to an antigen so as to shift the equilibrium of antigen and immunoglobulin single variable domain toward the presence of a complex formed by their binding. Thus, for example, where an antigen and antibody (fragment) are combined in relatively equal concentration, an antibody (fragment) of high affinity will bind to the available antigen so as to shift the equilibrium toward high concentration of the resulting complex. The dissociation constant is commonly used to describe the affinity between the antibody (fragment) and the antigenic target. Typically, the dissociation constant is lower than 10⁻⁵ M. Preferably, the dissociation constant is lower than 10⁻⁶ M, more preferably, lower than 10⁻⁷ M. Most preferably, the dissociation constant is lower than 10⁻⁸ M.

An immunoglobulin single variable domain that can specifically bind to and/or that has affinity for a specific antigen or antigenic determinant (e.g. epitope) is said to be "against" or "directed against" said antigen or antigenic determinant.

An immunoglobulin single variable domain according to the invention is said to be "cross-reactive" for two different antigens or antigenic determinants (such as urokinase plasminogen activator receptor from two different species of mammal, such as human uPAR and canine uPAR) if it is specific for both these different antigens or antigenic determinants.

It will be appreciated that, according to the invention, immunoglobulin single variable domains that are directed against the human uPAR may or may not show cross-reactivity with the uPAR from another species. For example, immunoglobulin single variable domains directed against human uPAR, in particular human uPAR (SEQ ID NO: 1) may or may not show cross-reactivity with uPAR from one or more other species of animals that are often used in animal models for diseases (for example, mouse, rat, rabbit, pig or dog).

Important to remark is that there is strong evidence for species specificity of uPAR, for instance showing that mouse uPA (or a fragment thereof) has a significantly reduced binding to human uPAR. As such, to preclinically investigate human uPAR-targeting agents, xenograft mouse models, having tumor tissue or cell lines from human propagated in ectopic or orthotopic sites in immunodeficient mice, are often used. However, xenograft mouse models are incapable of simulating the tumor microenvironment and cannot be used to study the interaction between tumors and host immunity because of the use of immunodeficient mice. Furthermore, it is becoming increasingly recognized that mice often poorly mimic human diseases, even when sophisticatedly manipulated with genetic techniques. An ideal animal model for cancer research should preferably be fully immunocompetent to properly mimic human immune responses.

Importantly, the immunoglobulin single variable domain of the invention shows cross-reactivity with canine urokinase plasminogen activator receptor (canine uPAR, SEQ ID NO: 10).

It will be clear to the skilled person that such cross-reactivity has advantages for diagnostic and/or therapeutic development, since it allows the immunoglobulin single variable domains of the invention to be tested in canine disease models.

Cancer in pet dogs is common and has been reported as a leading cause of death in aging dogs, accounting for greater than 1 in 4 deaths. As cancer in dogs occurs spontaneously and displays similar characteristics to many specific human tumor histologies, canine models are becoming more widely used in preclinical cancer research. Representative of this research opportunity, in 2003 the National Cancer Institute's (NCI) Center for Cancer Research established the Comparative Oncology Program to facilitate and support the design, sponsorship, and execution of translational trials in pet dogs to test novel anti-cancer drugs prior to human clinical trials. There are several advantages unique to canine models that were recognized and leveraged to expedite novel drug development ultimately slated for human usage. Because dogs are companion animals, they often live together with humans; therefore, they are exposed to the same environmental risk factors and might to a certain extent have a diet similar to humans. As with humans, a correlation between spontaneous tumor incidence and age is found in dogs. Additionally, from an evolutionary point of view, dogs are more closely related to humans than mice and share more similar physiologic and immunobiologic traits. Lastly, the high degree of homology in the human and canine genome makes analysis of DNA damage as well as epigenetic changes during tumor development and progression more facilely traceable and possible in outbred dogs.

Recently, several canine tumor histologies have been intensely studied using molecular cytogenetic techniques such as comparative genomic hybridization, oligonucleotide arrays, fluorescence in situ hybridization, and gene expression profiling. Based upon these genomic investigations, several conserved genetic similarities have been identified between canine and human tumors, including DNA copy number variations, structural chromosome aberrations, and differential gene expression patterns. These findings of shared genetic perturbations associated with distinct tumor histologies in both dogs and human beings further support the potential value of pet dogs with certain types of naturally occurring tumors as a unique model system for human-relevant cancer research. Importantly, canine tumors believed to be immunogenic including osteosarcoma, lymphoma, urothelial carcinoma, mammary gland carcinoma, melanoma, head and neck cancer and brain cancers have been the primary focus of most genomic based investigations.

As such, by using alternative animal models more closely related to humans, the success rate of oncology research and preclinical testing of anticancer therapies can be improved.

As such, the capability of the immunoglobulin single variable domain of the invention to target the canine homologue of uPAR, enables to better assess the biodistribution and other characteristics of the immunoglobulin single variable domain making use of more relevant larger animal models with spontaneous tumors. Canines display a high rate of spontaneous tumor formation and studies have shown that uPAR is expressed both in malignant epithelial cells and non-malignant stromal cells in the tumor microenvironment. Such a spontaneous tumor model, which has a more natural tumor microenvironment, is more accurate and informative as a disease model.

In addition, because of the capability of the immunoglobulin single variable domain of the invention to target both the canine and the human homologue of uPAR, there is no need to develop two different immunoglobulin single variable domains for preclinical and clinical studies, respectively, saving valuable time and money.

Furthermore, the capability of the immunoglobulin single variable domain of the invention to target the canine homologue of uPAR, enables the use of said immunoglobulin single variable domain in diagnosis, prognosis and/or treatment of cancer in canines.

In an embodiment, the invention relates to an immunoglobulin single variable domain that is directed against and/or that specifically binds to human urokinase plasminogen activator receptor (SEQ ID NO: 1), wherein the immunoglobulin single variable domain comprises an amino acid sequence that comprises 4 framework regions (FR) and 3 complementarity determining regions (CDR) according to the following formula (1):

FR1-CDR1-FR2-CDR2-FR3-CDR3-FR4 (1),

and wherein CDR1 is chosen from the group consisting of:
a. SEQ ID NO: 3,
b. Polypeptides that have at least 90% amino acid identity with SEQ ID NO: 3,
c. Polypeptides that have 1, 2 or 3 amino acid difference with SEQ ID NO: 3, and wherein CDR2 is chosen from the group consisting of:
   a. SEQ ID NO: 4,
   b. Polypeptides that have at least 90% amino acid identity with SEQ ID NO: 4,
   c. Polypeptides that have 1, 2 or 3 amino acid difference with SEQ ID NO: 4, and wherein CDR3 is chosen from the group consisting of:
      a. SEQ ID NO: 5,
      b. Polypeptides that have at least 90% amino acid identity with SEQ ID NO: 5,
      c. Polypeptides that have 1, 2 or 3 amino acid difference with SEQ ID NO: 5,
      More specifically, the framework regions (FRs) of the immunoglobulin single variable domains as described hereinabove have an amino acid sequence identity of more than 90% with the FRs of SEQ ID NO: 6 (FR1), SEQ ID NO: 7 (FR2), SEQ ID NO: 8 (FR3), SEQ ID NO: 9 (FR4).

In an embodiment, the invention relates to an immunoglobulin single variable domain that is directed against and/or that specifically binds to human urokinase plasminogen activator receptor (SEQ ID NO: 1), wherein the immunoglobulin single variable domain comprises an amino acid sequence that comprises 4 framework regions (FR) and 3 complementarity determining regions (CDR) according to the following formula (1):

FR1-CDR1-FR2-CDR2-FR3-CDR3-FR4 (1),

and wherein the complementarity determining regions (CDRs) comprises polypeptides that have at least 90% amino acid sequence identity with SEQ ID NO: 3 (CDR1), SEQ ID NO: 4 (CDR2), SEQ ID NO: 5 (CDR3).

In an embodiment, CDR1 comprises polypeptides that have more than 90%, preferably more than 91%, preferably more than 92%, preferably more than 93%, preferably more than 94%, preferably more than 95%, preferably more than 96%, preferably more than 97%, preferably more than 98%, preferably more than 99%, preferably 100% amino acid sequence identity with SEQ ID NO: 3. In an embodiment, CDR1 comprises polypeptides that have 1, 2 or 3 amino acid difference with SEQ ID NO: 3. In an embodiment, CDR2 comprises polypeptides that have more than 90%, preferably more than 91%, preferably more than 92%, preferably more than 93%, preferably more than 94%, preferably more than 95%, preferably more than 96%, preferably more than 97%, preferably more than 98%, preferably more than 99%, preferably 100% amino acid sequence identity with SEQ ID NO: 4. In an embodiment, CDR2 comprises polypeptides that have 1, 2 or 3 amino acid difference with SEQ ID NO: 4. In an embodiment, CDR3 comprises polypeptides that have more than 90%, preferably more than 91%, preferably more than 92%, preferably more than 93%, preferably more than 94%, preferably more than 95%, preferably more than 96%, preferably more than 97%, preferably more than 98%, preferably more than 99%, preferably 100% amino acid sequence identity with SEQ ID NO: 5. In an embodiment, CDR3 comprises polypeptides that have 1, 2 or 3 amino acid difference with SEQ ID NO: 5.

In an embodiment, the invention relates to an immunoglobulin single variable domain, wherein the framework regions (FRs) have an amino acid sequence identity of more than 90% with the FRs of SEQ ID NO: 6 (FR1), SEQ ID NO: 7 (FR2), SEQ ID NO: 8 (FR3), SEQ ID NO: 9 (FR4). In an embodiment, FR1 has an amino acid sequence identity of more than 90%, preferably more than 91%, preferably more than 92%, preferably more than 93%, preferably more than 94%, preferably more than 95%, preferably more than 96%, preferably more than 97%, preferably more than 98%, preferably more than 99%, preferably 100% with SEQ ID NO: 6. In an embodiment, FR1 comprises polypeptides that have 1, 2 or 3 amino acid difference with SEQ ID NO: 6. In an embodiment, FR2 has an amino acid sequence identity of more than 90%, preferably more than 91%, preferably more than 92%, preferably more than 93%, preferably more than 94%, preferably more than 95%, preferably more than 96%, preferably more than 97%, preferably more than 98%, preferably more than 99%, preferably 100% with SEQ ID NO: 7. In an embodiment, FR2 comprises polypeptides that have 1, 2 or 3 amino acid difference with SEQ ID NO: 7. In an embodiment, FR3 has an amino acid sequence identity of more than 90%, preferably more than 91%, preferably more than 92%, preferably more than 93%, preferably more than 94%, preferably more than 95%, preferably more than 96%, preferably more than 97%, preferably more than 98%, preferably more than 99%, preferably 100% with SEQ ID NO: 8. In an embodiment, FR3 comprises polypeptides that have 1, 2 or 3 amino acid difference with SEQ ID NO: 8. In an embodiment, FR4 has an amino acid sequence identity of more than 90%, preferably more than 91%, preferably more than 92%, preferably more than 93%, preferably more than 94%, preferably more than 95%, preferably more than 96%, preferably more than 97%, preferably more than 98%, preferably more than 99%, preferably 100% with SEQ ID NO: 9. In an embodiment, FR4 comprises polypeptides that have 1, 2 or 3 amino acid difference with SEQ ID NO: 9.

In an embodiment, the invention relates to an immunoglobulin single variable domain, wherein CDR1 consists of SEQ ID NO: 3. In an embodiment, the invention relates to an immunoglobulin single variable domain, wherein CDR2 consists of SEQ ID NO: 4. In an embodiment, the invention relates to an immunoglobulin single variable domain, wherein CDR3 consists of SEQ ID NO: 5. In an embodiment, the invention relates to an immunoglobulin single variable domain, wherein CDR1 consists of SEQ ID NO: 3 and CDR2 consists of SEQ ID NO: 4. In an embodiment, the invention relates to an immunoglobulin single variable domain, wherein CDR1 consists of SEQ ID NO: 3, CDR2 consists of SEQ ID NO: 4 and CDR3 consists of SEQ ID NO: 5. In an embodiment, the invention relates to an immunoglobulin single variable domain, wherein CDR1 consists of SEQ ID NO: 3 and CDR3 consists of SEQ ID NO: 5. In an embodiment, the invention relates to an immunoglobulin single variable domain, wherein CDR2 consists of SEQ ID NO: 4 and CDR3 consists of SEQ ID NO: 5.

In an embodiment, the invention relates to an immunoglobulin single variable domain, wherein FR1 consists of SEQ ID NO: 6. In an embodiment, the invention relates to an immunoglobulin single variable domain, wherein FR2 consists of SEQ ID NO: 7. In an embodiment, the invention relates to an immunoglobulin single variable domain, wherein FR3 consists of SEQ ID NO: 8. In an embodiment, the invention relates to an immunoglobulin single variable domain, wherein FR4 consists of SEQ ID NO: 9. In an embodiment, the invention relates to an immunoglobulin single variable domain, wherein FR1 consists of SEQ ID NO: 6 and/or FR2 consists of SEQ ID NO: 7 and/or FR3 consists of SEQ ID NO: 8 and/or FR4 consists of SEQ ID NO: 9; and/or any combination thereof.

In an embodiment, the invention relates to an immunoglobulin single variable domain, wherein FR1 consists of SEQ ID NO: 6 and FR2 consists of SEQ ID NO: 7. In an embodiment, the invention relates to an immunoglobulin single variable domain, wherein FR1 consists of SEQ ID NO: 6, FR2 consists of SEQ ID NO: 7, FR3 consists of SEQ ID NO: 8. In an embodiment, the invention relates to an immunoglobulin single variable domain, wherein FR1 consists of SEQ ID NO: 6, FR2 consists of SEQ ID NO: 7, FR3 consists of SEQ ID NO: 8 and FR4 consists of SEQ ID NO: 9. In an embodiment, the invention relates to an immunoglobulin single variable domain, wherein FR1 consists of SEQ ID NO: 6 and FR3 consists of SEQ ID NO: 8. In an embodiment, the invention relates to an immunoglobulin single variable domain, wherein FR1 consists of SEQ ID NO: 6 and FR4 consists of SEQ ID NO: 9. In an embodiment, the invention relates to an immunoglobulin single variable domain, wherein FR1 consists of SEQ ID NO: 6, FR3 consists of SEQ ID NO: 8 and FR4 consists of SEQ ID NO: 9. In an embodiment, the invention relates to an immunoglobulin single variable domain, wherein FR2 consists of SEQ ID NO: 7 and FR3 consists of SEQ ID NO: 8. In an embodiment, the invention relates to an immunoglobulin single variable domain, wherein FR2 consists of SEQ ID NO: 7 and FR4 consists of SEQ ID NO: 9. In an embodiment, the invention relates to an immunoglobulin single variable domain, wherein FR3 consists of SEQ ID NO: 8 and FR4 consists of SEQ ID NO: 9.

In an embodiment, the invention relates to an immunoglobulin single variable domain, wherein CDR1 consists of SEQ ID NO: 3 and/or CDR2 consists of SEQ ID NO: 4 and/or CDR3 consists of SEQ ID NO: 5; and/or wherein the framework regions (FRs) have an amino acid sequence of SEQ ID NO: 6 (FR1) and/or SEQ ID NO: 7 (FR2) and/or SEQ ID NO: 8 (FR3) and/or SEQ ID NO: 9 (FR4) and/or any combination thereof.

In an embodiment, the invention relates to an immunoglobulin single variable domain, wherein CDR1 consists of SEQ ID NO: 3, CDR2 consists of SEQ ID NO: 4, and CDR3 consists of SEQ ID NO: 5; and wherein the framework regions (FRs) have an amino acid sequence of SEQ ID NO: 6 (FR1), SEQ ID NO: 7 (FR2), SEQ ID NO: 8 (FR3), SEQ ID NO: 9 (FR4).

In an embodiment, the invention relates to an immunoglobulin single variable domain, wherein the immunoglobulin single variable domain comprising an amino acid sequence according to SEQ ID NO: 2.

Non-limiting examples of immunoglobulin single variable domains according to the present invention are as described herein and include a cross-reactive anti-human/anti-canine uPAR single domain antibody (sdAb), in particular SEQ ID NO: 2. In a specific embodiment, the sdAbs of the present invention may comprise at least one of the complementarity determining regions (CDRs) as described herein, for example CDRs with an amino acid sequence selected from SEQ ID NOs: 3-5. Preferably, the sdAbs of the present invention comprise a CDR1, a CDR2 and a CDR3 selected from the group consisting of SEQ ID NOs: 3-5, according to the above described formula (1). Preferably, a sdAb is provided comprising an amino acid sequence according to formula (1) with a CDR1 consisting of SEQ ID NO: 3, a CDR2 consisting of SEQ ID NO: 4, a CDR3 consisting of SEQ ID NO: 5, or with polypeptides that have at least 90% amino acid identity with SEQ ID NO: 3, SEQ ID NO: 4, SEQ ID NO: 5. More specifically, the sdAbs can comprise SEQ ID NO: 2, or a functional fragment thereof. A "functional fragment" or a "suitable fragment", as used herein, may for example comprise one of the CDR loops.

Also within the scope of the invention are natural or synthetic analogs, mutants, variants, alleles, homologs and orthologs (herein collectively referred to as "variants") of the immunoglobulin single variable domains of the invention as defined herein. Thus, according to one embodiment of the invention, the term "immunoglobulin single variable domain of the invention" in its broadest sense also covers such variants, in particular variants of the sdAb of SEQ ID NO: 2. Generally, in such variants, one or more amino acid residues may have been replaced, deleted and/or added, compared to the sdAbs of the invention as defined herein. Such substitutions, insertions or deletions may be made in one or more of the framework regions and/or in one or more of the CDR's, and in particular variants of the CDR's of the sdAbs of SEQ ID NO: 2, said CDR's corresponding to SEQ ID NO: 3 (CDR1), SEQ ID NO: 4 (CDR2), SEQ ID NO: 5 (CDR3). Variants, as used herein, are sequences wherein each or any framework region and each or any complementarity determining region shows at least 80% identity, preferably at least 85% identity, more preferably 90% identity, even more preferably 95% identity or, still even more preferably 99% identity with the corresponding region in the reference sequence, as can be measured electronically by making use of algorithms such as PILEUP and BLAST (Altschul et al. 1990, J Mol Biol 215: 403; Higgins & Sharp 1989, CABIOS 5: 151). Software for performing BLAST analyses is publicly available through the National Center for Biotechnology Information (http://www/ncbi.nlm.nih.gov/). Such variants of immunoglobulin single variable domains may be of particular advantage since they may have improved potency or other desired properties.

By means of non-limiting examples, a substitution may for example be a conservative substitution (as described herein) and/or an amino acid residue may be replaced by another amino acid residue that naturally occurs at the same position in another V_{H}H domain. Thus, any one or more substitutions, deletions or insertions, or any combination thereof, that either improve the properties of the sdAb of the invention or that at least do not detract too much from the desired properties or from the balance or combination of desired properties of the sdAb of the invention (i.e. to the extent that the sdAb is no longer suited for its intended use) are included within the scope of the invention. A skilled person will generally be able to determine and select suitable substitutions, deletions or insertions, or suitable combinations of thereof, based on the disclosure herein and optionally after a limited degree of routine experimentation, which may for example involve introducing a limited number of possible substitutions and determining their influence on the properties of the sdAb thus obtained.

According to particularly preferred embodiments, variants of the immunoglobulin single variable domains, in particular the sdAb of the present invention may have a substitution, deletion or insertion, of 1, 2 or 3 amino acids in one, two or three of the CDRs, more specifically (i) in CDR1 or CDR2 or CDR3; (ii) in CDR1 and CDR2, or, in CDR1 and CDR3, or, in CDR2 and CDR3; (iii) in CDR1 and CDR2 and CDR3. More preferably, variants of the immunoglobulin single variable domains, in particular the sdAbs, of the present invention may have a conservative substitution (as defined herein) of 1, 2 or 3 amino acids in one, two or three of the CDRs, more specifically (i) in CDR1 or CDR2 or CDR3; (ii) in CDR1 and CDR2, or, in CDR1 and CDR3, or, in CDR2 and CDR3; (iii) in CDR1 and CDR2 and CDR3.

Further, depending on the host organism used to express the immunoglobulin single variable domain of the invention, such deletions and/or substitutions may be designed in such a way that one or more sites for post-translational modification (such as one or more glycosylation sites) are removed, as will be within the ability of the person skilled in the art. Alternatively, substitutions or insertions may be designed so as to introduce one or more sites for attachment of functional groups (as described herein), for example to allow site-specific pegylation.

Examples of modifications, as well as examples of amino acid residues within the immunoglobulin single variable domain, preferably the sdAb sequence, that can be modified (i.e. either on the protein backbone but preferably on a side chain), methods and techniques that can be used to introduce such modifications and the potential uses and advantages of such modifications will be clear to the skilled person. Examples are discussed above.

Other cross-reactive anti-human/anti-canine uPAR immunoglobulin single variable domains, for example those having SEQ ID NOs: 20-22 (referred to in the examples as "sdAb16", "sdAb17", "sdAb18", respectively), show a less favourable pharmacokinetic profile, exhibiting for instance a relatively high unspecific background signal (as evidenced by the examples of the current application).

In an embodiment, the immunoglobulin single variable domain of the invention as described in any of the previous embodiments directed against and/or specifically binding to uPAR (for instance having SEQ ID NO: 2 or an immunoglobulin single variable domain comprising an amino acid sequence consisting of polypeptides that have at least 90% amino acid identity with SEQ ID NO: 2 or consisting of polypeptides that have 1, 2 or 3 amino acid difference with SEQ ID NO: 2 or an immunoglobulin single variable domain comprising at least one of the complementarity determining regions (CDRs) or framework regions (FRs) as described herein, for example CDRs with an amino acid sequence selected from SEQ ID NOs: 3-5 or FRs with an amino acid sequence selected from SEQ ID NOs: 6-9 or variants thereof) is linked with a tag, for instance to allow detection (for instance molecular imaging), targeted therapy, easy purification or site-specific labeling. Suitable tags are known from the art and include for instance affinity tags (such as Glutathione-S-transferase-tag, PolyHis tag, Biotin tag, Strep-tag, sortase-tag, glutamine-tag, unnatural amino acid tag, glycosylation-tag, etc.), fluorescent tags and epitope tags (such as C-myc-tag, Human influenza hemagglutinin (HA)-tag, DDDK-tag or V-tag). In an embodiment, the immunoglobulin single variable domain of the present invention can be linked to the tag either directly or through a linker. Linkers are described in more detail below.

In an embodiment, said immunoglobulin single variable domain of the invention directed against and/or specifically binding to uPAR is linked with a carboxy-terminal hexahistidine tag. In an embodiment, the immunoglobulin single variable domain having SEQ ID NO:2 (referred to in the examples as "sdAb15") is linked with a carboxy-terminal hexahistidine tag, said construct being referred to as "sdAb15.6His" in the examples. The underlying purification principle of the His-tag is an interaction between metal ions and the imidazole ring of histidine. In another embodiment, the immunoglobulin single variable domain of the invention directed against and/or specifically binding to uPAR is not linked with a tag. In said instance, the immunoglobulin single variable domain having SEQ ID NO:2 ("sdAb15") is also referred to as "sdAb15NT" ("NT"= "No Tag").

In an embodiment, the immunoglobulin single variable domain of the invention is fused to a one or more detectable labels or other signal-generating groups or moieties, depending on the intended use of the labelled immunoglobulin single variable domain. The immunoglobulin single variable domain of the invention can also be fused to a functional moiety, such as a therapeutically active agent.

Conjugates of the immunoglobulin single variable domain of the invention (or of a polypeptide comprising aforementioned immunoglobulin single variable domain), wherein the immunoglobulin single variable domain of the invention is fused to a detectable label or a functional moiety are further referred to as "conjugates of the invention".

Suitable labels and techniques for attaching, using and detecting them will be clear to the skilled person, and for example include, but are not limited to, fluorescent labels, phosphorescent labels, chemiluminescent labels or bioluminescent labels (such as luminal, isoluminol, theromatic acridinium ester, imidazole, acridinium salts, oxalate ester, dioxetane or GFP and its analogs), radio-isotopes, metals, metals chelates or metallic cations or other metals or metallic cations that are particularly suited for use in *in vivo, in vitro* or *in situ* diagnosis and imaging, as well as chromophores and enzymes (such as malate dehydrogenase, staphylococcal nuclease, delta- V- steroid isomerase, yeast alcohol dehydrogenase, alphaglycerophosphate dehydrogenase, triose phosphate isomerase, biotinavidin peroxidase, horseradish peroxidase, alkaline phosphatase, asparaginase, glucose oxidase, beta-galactosidase, ribonuclease, urease, catalase, glucose-VI- phosphate dehydrogenase, glucoamylase and acetylcholine esterase). In a preferred embodiment, the detectable label is chosen from the group of a radionuclide, a fluorescent moiety, a phosphorescent label, a chemiluminescent label, a metal, a metal chelate, a metallic cation, a chromophore, an enzyme or a combination of one of the aforementioned labels. Other suitable labels will be clear to the skilled person, and for example include moieties that can be detected using NMR or ESR spectroscopy. Such labelled immunoglobulin single variable domains and polypeptides of the invention may for example be used for *in vitro, in vivo* or *in situ* assays (including immunoassays known per se such as ELISA, RIA, EIA and other "sandwich assays", etc.) as well as *in vivo* diagnostic and imaging purposes, depending on the choice of the specific label.

As will be clear to the skilled person, another modification may involve the introduction of a chelating group, for example to chelate one of the metals or metallic cations referred to above. Suitable chelating groups for example include, without limitation, DTPA (Diethylentriaminepentaacetic acid) and derivatives (including 1B4M-DTPA derivatives and CHX-A"-DTPA derivatives), DOTA (1,4,7,10-Tetraazacyclododecane-1,4,7,10-tetraacetic acid) and derivatives (including DOTA-GA derivatives, DOTAM derivatives, DO3A and derivatives, DO2A and derivatives, CB-DO2A derivatives and DO3AM derivatives), NOTA (1,4,7-Triazacyclononane-1,4,7-triacetic acid) and derivatives (including NODA derivatives, NODA-GA derivatives, NO2A derivatives, NOTAM derivatives, NOPO derivatives and TRAP derivatives), HBED (N,N-bis(2-hydroxybenzyl)ethylenediamine-N,N-diacetic acid) and derivatives (including HBED-CC derivatives, HBED-CI derivatives, HBED-CA derivatives, HBED-AA derivatives and SHBED derivatives), DEPA (7-[2-(bis-carboxymethyl-amino)-ethyl]-4,10-bis-carboxymethyl-1,4,7,10-tetraaza-cyclododec-1-yl-acetic acid) and derivatives, picolinic acid (PA) based chelators and derivatives (including H2dedpa, H4octapa, H2azapa and H5decapa and derivatives), HEHA (1,2,7,10,13-hexaazacyclooctadecane-1,4,7,10,13,16-hexaacetic acid) and derivatives, TETA (1,4,8,11-tetraazacyclotetradecane-1,4,8,11-tetraacetic acid) and derivatives (including TE2A derivatives, CB-TE2A derivatives, CB-TE1A1P derivatives, CB-TE2P derivatives, MM-TE2A derivatives and DM-TE2A derivatives), NETA ([2-(4,7-bis-carboxymethyl-[1,4,7]triazacyclononan-1-yl-ethyl]-2-carbonylmethyl-amino]-tetraacetic acid) and derivatives (including C-NETA derivatives and NE3TA derivatives), AAZTA (1,4-bis(carboxymethyl)-6-[bis(carboxymethyl)]amino-6-methylperhydro-1,4-diazepine) and derivatives, DATA (6-amino-1,4-diazepine-triacetic acid) and derivatives, TCMC (1,4,7,10-tetraaza-1,4,7,10-tetra(2-carbamoylmethyl)cyclododecane) and derivatives, PCTA (3,6,9,15-tetraazabicyclo [9.3.1]pentadeca-1(15),11,13-triene-3,6,9-triacetic acid) and derivatives, Macropa (6-[[16-[(6-carboxypyridin-2-yl)methyl]-1,4,10,13-tetraoxa-7,16 diazacyclooctadec-7-yl]methyl]-4-isothiocyanatopyridine-2-carboxylic acid) and derivatives, THP (tris(hydroxypyridinone)) and derivates, DFO (deferoxamine) and derivatives, BCPA (N, N'-1,4-Butanediylbis[3-(2-chlorophenyl)acrylamide]) and derivatives, MAG-2 (2-Mercaptoacetyldiglycyl) and derivatives, MAG-3 (2-Mercaptoacetyltriglycyl) and derivatives, MAS-3 (mercaptoacetyltriserine) and derivatives, HYNIC (Hydrazinonicotinic acid) and derivatives and RESCA (Restrained Complexing Agent). In an embodiment, functional groups, such as maleimide, NCS and NHS, are added to the chelating agent in order to allow various conjugation methods. For instance, the isothiocyanate function (R-NCS) allows formation of stable thiourea bonds at alkaline pH with free amines. NHS is another example of an amine-reactive linker.

Yet another modification may comprise the introduction of a functional group that is one part of a specific binding pair, such as the biotin-(strept)avidin binding pair. Such a functional group may be used to link the immunoglobulin single variable domain of the invention to another protein, polypeptide or chemical compound that is bound to the other half of the binding pair, i.e. through formation of the binding pair. For example, an immunoglobulin single variable domain of the invention may be conjugated to biotin, and linked to another protein, polypeptide, compound or carrier conjugated to avidin or streptavidin. For example, such a conjugated immunoglobulin single variable domain may be used as a reporter, for example in a diagnostic system where a detectable signal-producing agent is conjugated to avidin or streptavidin. Such binding pairs may for example also be used to bind the immunoglobulin single variable domain of the invention to a carrier, including carriers suitable for pharmaceutical purposes. One non-limiting example are the liposomal formulations described by Cao and Suresh, Journal of Drug Targetting, 8, 4, 257 (2000). Such binding pairs may also be used to link a therapeutically active agent to the immunoglobulin single variable domain of the invention.

Thus, according to a preferred embodiment, the immunoglobulin single variable domain as used in the present invention is coupled or fused to a detectable label, either directly or through a linker. Preferably, the detectable label is a radio-isotope (radionuclide), in particular a radioactive tracer suitable for medical applications, such as in *in vivo* nuclear imaging. Examples include, without the purpose of being limitative, fluor 18 (¹⁸F), lutetium 177 (¹⁷⁷Lu), zirconium 89 (⁸⁹Zr), indium 111 (¹¹¹In), yttrium 90 (⁹⁰Y), copper 64 (⁶⁴Cu), gallium 67 (⁶⁷Ga), gallium 68 (⁶⁸Ga), technetium 99m (^{99m}Tc), iodium 123 (¹²³I), iodium 124 (¹²⁴I), iodium 125 (¹²⁵I), iodium 131 (¹³¹I), and any other radio-isotope which can be used in animals, in particular canine or human.

In another preferred embodiment, the detectable label is a fluorescent moiety. Examples include, without the purpose of being limitative, xanthene (e.g., fluorescein, rhodamine), cyanine (e.g., Cy5, Cy5.5, IRdye800CW etc), squaraines, dipyrromethene, bodipy and aza-bodipy, tetrapyrrole, naphthalene, oxadiazole, naphthalene, coumarin, oxazine derivatives and fluorescent metals such as europium or others metals from the lanthanide series.

For *in vivo* medical imaging research and practice, fluorophores operating in the NIR region are most often used, because of superior imaging characteristics compared to light in the visible spectrum. More specifically, the lower background and improved tissue penetration -a consequence of diminished scattering, endogenous fluorophore absorption and autofluorescence- makes NIR light more suited.

In the NIR region, a wide choice of fluorophores is available for conjugation. Appropriate fluorophores should be (photo)stable *in vitro* and *in vivo,* be soluble in aqueous environments, and their extinction coefficient and quantum yield should be as high as possible to maximize brightness. For the labeling of proteins and small molecule ligands for *in vivo* medical imaging, cyanine-based fluorescent dyes are by far the most widely investigated. Cyanine dyes typically consist of two heterocyclic nitrogen-containing rings connected by a polymethine bridge. Extension of the polymethine bridge to penta- and heptamethines, red-shifts the absorption and emission of the dyes. Dyes, e.g. IRDye800CW, have been rendered less hydrophobic by the inclusion of several charged groups such as sulfonic acids in their structure. Likewise, zwitterionic dyes such as ZW800-1, FNIR-Tag or s775z with balanced surface charges in their structure have been shown to interact very little with serum proteins, resulting in less background signals as compared to IRDye800CW-labeled tracers. In a preferred embodiment, FNIR-Tag is used as a detectable label. In another preferred embodiment, s775z is used as a detectable label.

While the use of NIR fluorescent dyes has led to increased signal-to-background ratios and better overall depth penetration up to several millimeter in tissue, deeper lying, and hidden lesions could still be missed, for instance in the field of cancer imaging. To this end, the combination of NIR fluorescence and nuclear techniques is attractive.

Combined use of radioactive and fluorescence signals can help strengthen *in vivo* medical imaging applications, such as image-guided surgery. This type of image guidance can come in 2 forms. In a first embodiment, separate radioactive and fluorescent tracers can be used, for instance for pre- and intraoperative imaging. To ensure surgical accuracy, in such a dual-tracer application one has to make sure both tracers independently allow delineation of the same lesions.

Said radioactive and fluorescent signature can also be integrated in a single bimodal/hybrid tracer. In an embodiment, said one or more detectable labels comprise a bimodal label comprising a fluorescent moiety and a radionuclide. Integration ensures colocalization of the two signatures and promotes an advanced form of symbiosis (the best of both worlds) that empowers surgeons to improve intraoperative target delineation. Hybrid tracers can come in many forms; not only can the biomolecule or targeting vehicle on which they are based vary, but they also may use different radionuclides (e.g., β or γ emission) or fluorescent moieties (e.g., light with different wavelengths). Although each individual hybrid tracer and administration route has been designed to serve a specific purpose, conceptually all use revolves around the notion that both signatures can be used to (for instance intraoperatively) depict complementary features of the same target. Despite differences in signal intensities, there is a high level of overlap in the way multiplexing of the different imaging signatures occurs. In the context of surgical guidance, the radioactive signal allows identification and localization of a lesion by means of its radioactive signature (even in deeper tissue layers), whereas the fluorescent signal allows direct lesion visualization and delineation in exposed tissue in the surgical field or provides high-resolution pathologic identification of the tracer accumulation.

In the field of cancer surgery, the nuclear component can be used for preoperative imaging, as well as for intraoperative guidance using a gamma-detecting probe (coarse navigation), at which point, fluorescence would provide high resolution visual guidance for precise resection. In these types of surgeries, the primary goal is to attain complete removal of all cancerous tissue and obtain negative tumor resection margins. By minimizing the risk of leaving cancer cells behind, chances of recurrence are diminished and overall survival is improved.

As such, in an embodiment, the radionuclide component of the conjugate of the invention is used for preoperative imaging. Such preoperative imaging allows to better plan the surgery as it provides useful information on the anatomical localization of the tumor lesions and presence of lymph node metastases and often involves SPECT or PET imaging. In another embodiment, the radionuclide component of the conjugate can provide additional guidance during the surgery itself or during *ex vivo* analysis of the resected tissues via gamma-ray, positron or beta-minus detection (probe or camera), or Cerenkov luminescence imaging. Subsequent intraoperative imaging allows *in situ* fluorescence-based visualization and fluorescence-guided removal of even submillimeter tumor lesions. Such a bimodal tracer allows detection of larger lesions via nuclear imaging, while fluorescence enables accurate removal of submillimeter lesions.

In another preferred embodiment, the immunoglobulin single variable domain as used in the present invention is coupled to or fused to a functional moiety, in particular a therapeutically active agent, either directly or through a linker. As discussed above, said immunoglobulin single variable domains as used in the present invention coupled to or fused to a functional moiety are also referred to as "conjugates of the invention".

As used herein, a "therapeutically active agent" means any molecule that has or may have a therapeutic effect (i.e. curative or stabilizing effect) in the context of treatment of a disease (as described further herein). Preferably, a therapeutically active agent is a disease-modifying agent, which can be a cytotoxic agent, such as a toxin, or a cytotoxic drug, or an enzyme capable of converting a prodrug into a cytotoxic drug, or a radionuclide, or a cytotoxic cell, or which can be a non-cytotoxic agent. Even more preferably, a therapeutically active agent has a curative effect on the disease.

As used herein, "linker molecules" or "linkers" are peptides of 1 to 200 amino acids length, and are typically, but not necessarily, chosen or designed to be unstructured and flexible. For instance, one can choose amino acids that form no particular secondary structure. Or, amino acids can be chosen so that they do not form a stable tertiary structure. Or, the amino acid linkers may form a random coil. Such linkers include, but are not limited to, synthetic peptides rich in Gly, Ser, Thr, Gin, Glu or further amino acids that are frequently associated with unstructured regions in natural proteins (Dosztanyi, Z., Csizmok, V., Tompa, P., & Simon, I. (2005). IUPred: web server for the prediction of intrinsically unstructured regions of proteins based on estimated energy content. Bioinformatics (Oxford, England), 21(16), 3433-4.).

Thus, according to specific embodiments, the amino acid (AA) linker sequence is a peptide of between 0 and 200 AA, between 0 and 150 AA, between 0 and 100 AA, between 0 and 90 AA, between 0 and 80 AA, between 0 and 70 AA, between 0 and 60 AA, between 0 and 50 AA, between 0 and 40 AA, between 0 and 30 amino acids, between 0 and 20 AA, between 0 and 10 amino acids, between 0 and 5 amino acids. Non-limiting examples of suitable linker sequences include (GS)5 (GSGSGSGSGS; SEQ ID NO: 11), (GS)10 (GSGSGSGSGSGSGSGSGSGS; SEQ ID NO: 12), (G4S)3 (GGGGSGGGGSGGGGS; SEQ ID NO: 13), llama IgG2 hinge (AHHSEDPSSKAPKAPMA; SEQ ID NO: 14) or human IgA hinge (SPSTPPTPSPSTPPAS; SEQ ID NO: 15) linkers. Examples of sequences of short linkers include, but are not limited to, PPP, PP or GS.

For certain applications, it may be advantageous that the linker molecule comprises or consists of one or more particular sequence motifs. For example, a proteolytic cleavage site can be introduced into the linker molecule such that detectable label or moiety can be released. Useful cleavage sites are known in the art, and include a protease cleavage site such as Factor Xa cleavage site having the sequence IEGR (SEQ ID NO: 16), the thrombin cleavage site having the sequence LVPR (SEQ ID NO: 17), the enterokinase cleaving site having the sequence DDDDK (SEQ ID NO: 18), or the PreScission cleavage site LEVLFQGP (SEQ ID NO: 19).

Alternatively, in case the immunoglobulin single variable domain is linked to a detectable label or moiety using chemoenzymatic methods for protein modification, the linker moiety may exist of different chemical entities, depending on the enzymes or the synthetic chemistry that is used to produce the covalently coupled molecule *in vivo* or *in vitro* (reviewed in: Rabuka 2010, Curr Opin Chem Biol 14: 790-796).

In a particular embodiment, the immunoglobulin single variable domains of the invention are in a "multivalent" form and are formed by bonding, chemically or by recombinant DNA techniques, together two or more monovalent immunoglobulin single variable domains. Non-limiting examples of multivalent constructs include "bivalent" constructs, "trivalent" constructs, "tetravalent" constructs, and so on. The immunoglobulin single variable domains comprised within a multivalent construct may be identical or different. In another particular embodiment, the immunoglobulin single variable domains of the invention are in a "multi-specific" form and are formed by bonding together two or more immunoglobulin single variable domains, of which at least one with a different specificity. Non- limiting examples of multi-specific constructs include "bi-specific" constructs, "tri-specific" constructs, "tetra-specific" constructs, and so on. To illustrate this further, any multivalent or multispecific (as defined herein) immunoglobulin single variable domain of the invention may be suitably directed against two or more different epitopes on the same antigen, for example against two or more different parts of uPAR; or may be directed against two or more different antigens, for example against uPAR and one or more other marker. Preferably, a monovalent immunoglobulin single variable domain of the invention is such that it will bind to the uPAR (as described herein) with an affinity less than 500 nM, preferably less than 200 nM, more preferably less than 10 nM, such as less than 500 pM. Multivalent or multispecific immunoglobulin single variable domains of the invention may also have (or be engineered and/or selected for) increased avidity and/or improved selectivity for the desired uPAR, and/or for any other desired property or combination of desired properties that may be obtained by the use of such multivalent or multispecific immunoglobulin single variable domains.

In a further aspect, the present invention also provides a polypeptide comprising any of the immunoglobulin single variable domains according to the invention, either in a monovalent, multivalent or multi-specific form. Thus, polypeptides comprising monovalent, multivalent or multi- specific immunoglobulin single variable domains are included here as non-limiting examples.

Another aspect of the invention relates to a nucleic acid sequence encoding an immunoglobulin single variable domain, in particular a single domain antibody fragment (sdAb), or a polypeptide of the invention, as described hereinbefore.

Further, the present invention also envisages expression vectors comprising nucleic acid sequences encoding any of the above immunoglobulin single variable domains or polypeptides, as well as host cells expressing such expression vectors. Suitable expression systems include constitutive and inducible expression systems in bacteria or yeasts, virus expression systems, such as baculovirus, semliki forest virus and lentiviruses, or transient transfection in insect or mammalian cells. Suitable host cells include E. coli, Lactococcus lactis, Saccharomyces cerevisiae, Schizosaccharomyces pombe, Pichia pastoris, and the like. Suitable animal host cells include HEK 293, COS, S2, CHO, NSO, DT40 and the like. The cloning, expression and/or purification of the immunoglobulin single variable domain can be done according to techniques known by the skilled person in the art.

The invention also relates to a method for producing aforementioned immunoglobulin single variable domain or aforementioned polypeptide, said method comprising the steps of:
- expressing, in a suitable host cell or a suitable expression system, aforementioned nucleic acid sequence; and optionally
- isolating and/or purifying aforementioned immunoglobulin single variable domain or aforementioned polypeptide.

In still another aspect, the invention also relates to a pharmaceutical composition comprising an immunoglobulin single variable domain of the invention (or conjugate of the present invention), and at least one of a pharmaceutically acceptable carrier, adjuvant or diluent.

The pharmaceutical compositions of the present invention can be administered by any appropriate route. Suitable routes of administration include, but are not limited to, orally, parenterally, intravenously, intradermally, intramuscularly or subcutaneously, rectally, intraperitoneally, via inhalation or via buccal administration, via sublingual route, or topically e.g. transdermally.

In some embodiments, the pharmaceutical composition can be administered through urogenital routes to reach to targets, such as internal organs, topical lesions, or organs can be accessed by instillation (such as urinary bladder, vaginal cannel) more effectively and efficiently.

In some embodiments, the pharmaceutical compositions of the invention contain a pharmaceutically acceptable excipient suitable for rendering the composition administrable orally, parenterally, intravenously, intradermally, intramuscularly or subcutaneously, rectally, intraperitoneally, via inhalation or via buccal administration, via sublingual route, or topically e.g. transdermally.

The immunoglobulin single variable domains of the invention (or conjugates of the invention) can be admixed or compounded with a conventional, pharmaceutically acceptable excipient. It will be understood by those skilled in the art that a mode of administration, vehicle, excipient or carrier conventionally employed and which is inert with respect to the immunoglobulin single variable domain or conjugate of the invention can be utilized for preparing and administering the pharmaceutical compositions of the present invention. The formulations of the present invention for use in a subject comprise the immunoglobulin single variable domain of the invention (or conjugate of the present invention), together with one or more acceptable excipients, and optionally therapeutic agents. The excipient must be "acceptable" in the sense of being compatible with the other ingredients of the formulation and not deleterious to the recipient thereof.

The pharmaceutical composition may be conveniently made available in a unit dosage form, whereby such formulations may be prepared by any of the methods generally known in the pharmaceutical arts. The combination of the immunoglobulin single variable domain (or conjugate) of the present invention with the one or more adjuvants is then physically treated to present the formulation in a suitable form for delivery (e.g., forming an aqueous suspension, by entrapping the drug in liposomes or microemulsions which are compatible with body tissues).

Adjuvants or accessory ingredients for use in the formulations of the present invention can include any pharmaceutical ingredient commonly deemed acceptable in the art, such as mixtures, buffers, solubility enhancers, and the like.

Since administration of parenteral dosage forms typically bypasses the patient's natural defenses against contaminants, parenteral dosage forms can be sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms of the compounds of the invention include, without limitation: sterile water; water for injection USP; saline solution; phosphate buffered saline; glucose solution; aqueous vehicles such as but not limited to, sodium chloride injection, Ringer's injection, dextrose Injection, dextrose and sodium chloride injection, and lactated Ringer's injection; water-miscible vehicles such as, but not limited to, ethyl alcohol, polyethylene glycol, and propylene glycol; and non-aqueous vehicles such as, but not limited to, corn oil, cottonseed oil, peanut oil, sesame oil, ethyl oleate, isopropyl myristate, and benzyl benzoate. Compounds that alter or modify the solubility of a pharmaceutically acceptable salt of a compound of the invention as disclosed herein can also be incorporated into the parenteral dosage forms of the disclosure, including conventional and controlled-release parenteral dosage forms.

Formulations for parenteral administration include aqueous and non-aqueous sterile injection solutions, which may further contain additional agents, such as antioxidants, buffers, bacteriostats, and solutes, which render the formulations isotonic with the blood of the intended recipient. In an embodiment, the composition comprises additives that reduce unwanted retention of the immunoglobulin single variable domain of the invention (or conjugate of the present invention) in the body, such as positively charged amino acids that have been shown to reduce kidney retention. The formulations may include aqueous and non-aqueous sterile suspensions, which contain suspending agents and thickening agents.

Injectable preparations, for example, sterile injectable aqueous or oleaginous suspensions can be formulated according to the known art using suitable dispersing or wetting agents and suspending agents. The sterile injectable preparation can also be a sterile injectable solution, suspension or emulsion in a nontoxic parenterally acceptable diluent or solvent, for example, as a solution in 1,3-butanediol. Among the acceptable vehicles and solvents that can be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable media prior to use.

The present invention also provides convenient pharmaceutical kits. Such kits can comprise the immunoglobulin single variable domain of the invention (or conjugate of the present invention) and, typically, a pharmaceutically acceptable carrier. The kit can also further comprise conventional kit components, such as needles for use in injecting the compositions, one or more vials for mixing the composition components, and the like, as are apparent to those skilled in the art. In addition, instructions, either as inserts or as labels, indicating quantities of the components, guidelines for mixing the components, and protocols for administration, can be included in the kits.

Preferably, the pharmaceutical composition comprises a therapeutically effective amount (or an amount effective for diagnosis/prognosis) of an immunoglobulin single variable domain or conjugate of the invention, and at least one of a pharmaceutically acceptable carrier, adjuvant or diluent.

A 'carrier', or 'adjuvant', in particular a 'pharmaceutically acceptable carrier' or 'pharmaceutically acceptable adjuvant' is any suitable excipient, diluent, carrier and/or adjuvant which, by themselves, do not induce the production of antibodies harmful to the individual receiving the composition nor do they elicit protection. So, pharmaceutically acceptable carriers are inherently non-toxic and nontherapeutic, and they are known to the person skilled in the art. Suitable carriers or adjuvantia typically comprise one or more of the compounds included in the following nonexhaustive list: large slowly metabolized macromolecules such as proteins, polysaccharides, polylactic acids, polyglycolic acids, polymeric amino acids, amino acid copolymers and inactive virus particles. Carriers or adjuvants may be, as a non limiting example, Ringer's solution, dextrose solution or Hank's solution. Non aqueous solutions such as fixed oils and ethyl oleate may also be used. A preferred excipient is 5% dextrose in saline. The excipient may contain minor amounts of additives such as substances that enhance isotonicity and chemical stability, including buffers and preservatives.

As used herein, the terms "therapeutically effective amount", "therapeutically effective dose" and "effective amount" mean the amount needed to achieve the desired result or results. As used herein, "pharmaceutically acceptable" means a material that is not biologically or otherwise undesirable, i.e., the material may be administered to an individual along with the compound without causing any undesirable biological effects or interacting in a deleterious manner with any of the other components of the pharmaceutical composition in which it is contained.

In a further aspect, the invention relates to aforementioned immunoglobulin single variable domain or aforementioned polypeptide, wherein the immunoglobulin single variable domain specifically targets human and/or canine uPAR-positive cells.

By specifically targeting uPAR-positive cells, the immunoglobulin single variable domain of the invention can, when coupled to a detectable label or a functional moiety, visualize uPAR-positive cells or target therapeutically active agents to said uPAR-positive cells, respectively.

uPAR mediates a wide variety of cellular processes including inflammation, metastasis and invasion, tissue remodeling, angiogenesis, and cell adhesion. By specifically targeting uPAR-positive cells, the immunoglobulin single variable domain of the invention can help in visualizing said processes or help in therapeutically targeting the cells involved in said processes.

For instance, as uPAR shows increased expression in many human tumors, both on the cancer cells themselves as well as on stromal cells, the immunoglobulin single variable domain of the invention coupled or fused to a detectable label can help in delineating the tumor and his micro-environment. Likewise, the immunoglobulin single variable domain of the invention coupled or fused to a functional moiety, can help in specifically targeting compounds of interest to said tumor-associated cells and as such assist in cancer therapy.

By visualizing or detecting uPAR-positive cells the conjugates of the invention can assist in non-invasive *in vivo* medical imaging.

As such, the invention further relates to aforementioned immunoglobulin single variable domain or aforementioned polypeptide for use as contrast agent in noninvasive *in vivo* imaging, such as molecular imaging.

Molecular imaging has been defined as "the visualization, characterization, and measurement of biological processes at the molecular and cellular level in living subjects". The technique makes use of molecular tracers seeking for biomarkers expressed in (patho)physiological processes. Molecular tracers most often consist of a targeting moiety to direct the tracer and a signaling moiety for detection. Following administration, the molecular tracer will specifically accumulate in areas where the biomarker reveals itself, while unbound tracer will be eliminated. The bound molecular tracer is then visualized by means of an appropriate imaging modality.

Radioactivity and fluorescence are particularly suited for the application because of their high detection sensitivity. Yet, the choice of detection system and associated signaling molecule is directly linked to the intended application. In the clinic, nuclear molecular imaging can be used as a noninvasive technique for the diagnosis of diseases, early patient stratification according to the expression of predictive biomarkers, or treatment follow-up and response prognosis. Alternatively, as fluorescence signals have limited depth penetration (several mm at max), the use of fluorescence imaging is restricted to imaging of accessible tissues (e.g. skin surface, oral mucosa) and as tool during interventional procedures, i.e., surgery, endoscopic, and intravascular imaging. Over the last decade, fluorescence-guided interventions have gained interest as a method to assist surgeons in real-time by demarcating cancerous tissues for precise and complete resection, by highlighting healthy tissues that should be preserved, by guiding biopsy, or by interrogating suspicious lesions *in vivo* at the molecular level.

*In vivo* imaging may be used to provide 2-D as well as 3-D images of a mammal. Charge-coupled device cameras, photodiodes, avalanche photodiodes, photomultiplier tubes, CMOS, or 3D tomographers may used to carry out *in vivo* imaging. In some embodiments, *in vivo* imaging comprises optical imaging and may be performed by detecting a label that emits light at a wavelength designed to penetrate living tissue. Such labels include long wavelength emitting fluorescent dyes or proteins such as infrared and near infrared dyes or proteins including but not limited to dyes or proteins that emit in the range of about 600 nm to about 800 nm, about 650 nm to about 800 nm, or about 700 nm to about 800 nm. Alternatively, labels designed to emit light that penetrates living tissue may include non fluorescent reagents including but not limited to red-shifted luciferases.

*In vivo* imaging can also involve computed tomography, magnetic resonance imaging, ultrasonography, positron emission tomography, single-photon emission computed tomography (SPECT), etc. SPECT can also be used with an integrated x-ray CAT (CT) scanner (SPECT/CT). The information from many *in vivo* imaging methods as those described above can provide 3D distribution of the immunoglobulin single variable domains of the current invention (an hence the uPAR-positive cells) in the subject.

In an embodiment, conjugates of the invention, comprising the immunoglobulin single variable domain of the invention (or a polypeptide comprising said immunoglobulin single variable domain) and a detectable label (more specifically a fluorescent moiety) are used in intraoperative image-guided surgery.

Image-guide surgery is a technology where the surgeon intra-operatively is guided by imaging of the tumor. Near-infrared fluorescent ligands are mostly used because their wavelength is invisible to the human eye and therefore does not cause any interference with visualization of the operating field. Usage of fluorescence imaging during surgery provides a real-time, sensitive, contact-free, relatively cheap, and non-ionizing technique that can easily be implemented within the surgical routine. Fluorescence imaging can provide anatomical, functional, or molecular information after administration of a fluorescent contrast agent, either through direct real-time imaging of the surgical field, or by intraoperative optical specimen mapping. Imageguidance help to make surgeries less invasive and more precise, which can lead to better survival, less complications, less morbidity, better quality of life, shorter hospital stays and fewer repeated procedures.

In an embodiment, aforementioned conjugate of the invention is used in intraoperative image-guided surgery in cancer therapy. Surgery, in combination with or without chemo and/or radiotherapy, remains the most recommended treatment with curative intent for many localized tumors. In these types of surgeries, the primary goal is to attain complete removal of all cancerous tissue and obtain negative tumor resection margins. By minimizing the risk of leaving cancer cells behind, chances of recurrence are diminished and overall survival is improved. As visual inspection through the surgeon's eyes (open surgeries) or through color video (laparoscopic interventions) is often insufficient. The conjugate according to the current invention allows for real-time and sensitive information regarding the location of tumor lesions, allowing the detection of occult tumor lesions, accurate and real-time definition of tumor margins, and assessment of the presence of locoregional LN metastases. As such, the conjugate according to the current invention allows for complete removal of all cancerous tissue and obtaining negative tumor resection margins, a major step forward in preventing over- and undertreatment, and personalizing the surgical treatment of many cancer patients.

Total removal of tumor tissue during curative-intended cancer surgery is pivotal. However, the correct delineation of tumor tissue from normal tissue is often difficult. As uPAR is expressed along the invasive front of multiple tumors, uPAR-targeted ligands for optical imaging allow for establishing valid resection margins during surgery, which potentially could reduce the number of patients with postoperative recurrences of cancer.

In an embodiment, the use of the conjugate or composition of the current invention is used in preoperative imaging. Preoperative imaging findings support lesion identification during the intervention. The value of knowing where lesions are located during surgical procedures in relation to the patient's anatomic context is especially eminent when resections are performed in complex anatomies (e.g., the pelvic area).

In an embodiment of the invention, aforementioned conjugate or composition is used in endoscopic or intravascular imaging. Such imaging techniques can for instance be used for an improved prognosis and diagnosis of a disease or pathology.

In an embodiment, aforementioned conjugate or composition is used in endoscopic imaging for the detection of cancerous or precancerous lesions. White light endoscopy (WLE) has been the gold standard for the detection of lesions in the gastrointestinal tract. Despite the efficacy of current endoscopy, small and inconspicuous lesions or serrated adenomas tend to be flat and do not exhibit detectable discoloration relative to normal tissue in the three reflectance color channels (red/green/blue, RGB) used in WLE. This makes the detection of disease and the acquisition of targeted biopsies with conventional WLE a challenging issue. The use of the conjugate or composition of the current invention allows to detect inflammatory or (pre)cancerous lesions that might not have been detected using WLE, permitting more targeted biopsies and improving diagnostic performance.

In a further aspect, the invention relates to aforementioned immunoglobulin single variable domain or aforementioned polypeptide for use in diagnosis, prognosis and/or treatment of cancer.

As described above, uPAR shows increased expression in many human tumors, both on the cancer cells themselves as well as on stromal cells. As such, the immunoglobulin single variable domain of the invention coupled or fused to a detectable label can help in delineating the tumor and his micro-environment and as such assist in diagnosis and prognosis of cancer and choice of therapy. uPAR expression is increased in many human cancers and correlates with a poor prognosis and early invasion and metastasis. Likewise, the immunoglobulin single variable domain of the invention coupled or fused to a functional moiety, can help in specifically targeting compounds of interest to said tumor-involved cells and as such assist in cancer treatment.

### The immunoglobulin single variable domain or aforementioned polypeptide for use in diagnosis, prognosis and choice of therapy.

The present disclosure enables the detection of human (and canine) uPAR in a biological sample *in situ* or isolated from a subject. Since certain cancers (e.g. metastatic cancer) overexpress uPAR, detection of uPAR can aid in diagnosis, choice of therapy, prognosis and follow-up of disease. As the conjugate or composition of the current invention (comprising the immunoglobulin single variable domain of the invention) allows for an improved pharmacokinetic profile compared to other tracers currently known in the field of medical imaging, a more accurate diagnosis, prognosis and choice of therapy can be obtained by the use of the conjugate of the current invention.

In the context of the present invention, prognosing an individual suffering from or suspected to suffer from cancer refers to a prediction of the survival probability of an individual having cancer or prediction of the relapse risk which is related to the invasive or metastatic behavior (i.e. malignant progression) of tumor tissue or cells. In an embodiment, said prognosis comprises cancer staging. Cancer staging is determined based on the size of the cancer and if or to what extent the cancer has spread to other parts of the patient's body. The conjugate of the current invention allows for an improved pharmacokinetic profile compared to other tracers currently known in the field of medical imaging. As such, a more accurate prognosis (including a more accurate cancer stage) can be determined. This allows to identify the most optimal treatment (for instance certain ongoing clinical trials) and improves the disease outcome (e.g. death, chance of recovery, recurrence).

Detecting human (or canine) uPAR in a biological sample containing a cell involves contacting the sample with the conjugate of the invention; and directly or indirectly detecting binding of the conjugate of the invention to a cell in the sample.

The cell can be *in vitro,* where the cell is in a biological sample obtained from a patient suspected of having, or known to have uPAR-positive cells (e.g. cancer cells), a patient undergoing treatment, or a patient being tested for susceptibility to treatment. The cell can be *in vivo,* e.g., the cell is in a patient suspected for having cancer cells, a patient undergoing treatment, or a patient being tested for susceptibility to treatment.

The conjugates of the invention can be used to detect uPAR-expressing cells in a biological sample of a subject having or suspected of having cells expressing a detectable level of uPAR (e.g. cancer cells) using immunodiagnostic techniques known in the art. Some examples of cancer cells that can be detected using the immunoglobulin single variable domains of the invention include cancer cells of the breast. An example of a breast cancer cell that can be detected includes triple negative cancer cells that are negative for Her2/neu, estrogen receptor, and progesterone receptor (Pal S K et al. (2009) Maturitas 63:269-274; Ahmad A et al. (2009) Journal of Cellular Biochemistry 108:916-925). Other cancers that can be detected include cancers in the ovaries, prostate, testes, colon, rectum, lung, brain, blood, bone, marrow, or any other organ or tissue in the body, including but not limited to leukaemias, fibrosarcomas, and glioblastomas. Such diagnostics can be useful to identify patients amenable to the therapies disclosed herein, and/or to monitor response to therapy.

Suitable immunodiagnostic techniques include, but are not necessarily limited to, both *in vitro* and *in vivo* methods (e.g. imaging).

Where the cell detected using the subject method is *in vivo,* the method can determine the presence or absence of a particular uPAR-positive cell and/or the location of the uPAR-positive cell in a patient. For example, the conjugates of the invention can help determine if a cancer cell positive for uPAR has migrated away from the original tumor, the presence or absence of a cancer cell positive for uPAR in the lymph nodes, and/or can help identify lymph nodes containing uPAR-positive cells.

The invention also relates to aforementioned immunoglobulin single variable domain or aforementioned polypeptide for use in monitoring cancer therapy.

In some embodiments, immunoglobulin single variable domains suitable for the purpose of detection of uPAR-expressing cancer cells can be used to track the progress of anti-cancer treatments including anti-cancer treatments directed toward uPAR positive cancer cells, for example by detecting any decrease or increase in tumor size *in vivo.*

Conjugates of the invention can be administered via local injection, e.g. at a tumor site or site suspected of having cells expressing uPAR, or systemically including but not limited to infusion (e.g. arterial or venous infusion), or injection (e.g. intravenous, intraarterial, intrathecal, intreacranial, subcutaneous, intramuscular, or other method of injection known in the art).

Where the methods are *in vitro,* the biological sample can be any sample in which uPAR may be present, including but not limited to tissues, whole cells, and extracts thereof. For example, the assay can involve detection of uPAR on cells in a histological tissue sample. For example, the tissue sample may be fixed (e.g., by formalin treatment) and may be provided embedded in a support (e.g., in paraffin) or frozen unfixed tissue. Assays can take a wide variety of forms, such as competition, direct reaction, or sandwich type assays. Examples of assays include Western blots; agglutination tests; enzyme-labeled and mediated immunoassays, such as enzyme-linked immunosorbent assays (ELISAs); biotin/avidin type assays; radioimmunoassays; immunoelectrophoresis; immunoprecipitation, fluorescence activated cell sorting, and the like. The reactions generally include detectable labels such as fluorescent, chemiluminescent, radioactive, enzymatic labels or dye molecules, or other methods for detecting the formation of a complex between antigen in the sample and the immunoglobulin single variable domain reacted therewith.

Diagnostic assays can also be conducted *in situ.* For example, anti-uPAR immunoglobulin single variable domains of the invention can be detectably labeled (forming conjugates of the invention), administered to a subject suspected of having a cancer characterized by cell surface expression of uPAR, and bound conjugate can be detected using imaging methods available in the art, including but not limited to those *in vivo* imaging methods described herein.

For instance, the diagnostic assays described herein can be used to determine whether a subject has a cancer that is more or less amenable to therapy using antibody-based therapy, as well as monitor the progress of treatment in a subject. It also may be used to assess the course of other combination therapies. Thus, the diagnostic assays can inform selection of therapy and treatment regimen by a clinician.

In an embodiment, aforementioned conjugate or composition is used in image-guided therapy of a disease or pathology. Image-guided therapy refers to the use of any form of medical imaging to plan, perform, and evaluate surgical procedures and therapeutic interventions. While the number of specific procedures that use imageguidance is growing, these procedures comprise two general categories: traditional surgeries that become more precise through the use of imaging and newer procedures that use imaging and special instruments to treat conditions of internal organs and tissues via minimally invasive surgery. The most commonly used modalities of image-guided therapy include Magnetic Resonance Imaging (MRI), Computed Tomography (CT), nuclear imaging and optical imaging using fluorescent tracers. Image-guided therapy can also be supported by ultrasound, angiography, surgical navigation equipment, tracking tools, and integration software.

In an embodiment, aforementioned conjugate or composition is used in pre- and posttherapy assessment.

In another embodiment, aforementioned conjugate or composition is used in nonsurgical image-guided therapy, such as thermal ablation therapy. Ablation therapy relies on the principle that the tumor is not removed (like in a surgical resection) but rather destroyed in place by elevating the temperature within the tumorous tissues above a lethal threshold. The conjugate or composition of the current invention allows to accurately localize the tumor tissues that need to be ablated. Minimally invasive thermal ablation procedures are particularly well tolerated by the patients and require reduced hospital stays. In an embodiment, these image-guided ablation devices can be based on focused ultrasound (also called FUS or HIFU).

As the PA system has also been shown to have a key role in the pathogenesis of vascular diseases (including atherosclerosis, thromboembolic disorders and stroke), inflammatory diseases (such as inflammatory bowel disease, rheumatoid arthritis) or wound healing, the conjugates of the invention could also be used as an uPAR-targeting agent in said diseases, for instance for the delivery of therapeutic drugs to the site of interest.

The above-described assay reagents, including the immunoglobulin single variable domain of the invention, can be provided in kits, with suitable instructions and other necessary reagents, in order to conduct immunoassays as described above. The kit can also contain, depending on the particular immunoassay used, suitable labels and other packaged reagents and materials (i.e. wash buffers and the like). Standard immunoassays, such as those described above, can be conducted using these kits.

### The immunoglobulin single variable domain or aforementioned polypeptide for use in cancer treatment.

The uPAR-binding immunoglobulin single variable domains of the present disclosure can also find use as therapeutic for treatment of proliferative disorders that are mediated by uPAR-expressing cells. For example, the uPAR-binding immunoglobulin single variable domains of the present disclosure can be used in a therapy for a uPAR-expressing cancer (including prevention and post-diagnosis therapy). Subjects having, suspected of having, or at risk of developing a uPAR-expressing cancer are contemplated for therapy described herein.

In an embodiment, the immunoglobulin single variable domain as used in the present invention for us in cancer treatment is coupled to or fused to a functional moiety, in particular a therapeutically active agent, either directly or through a linker.

As used herein, a "therapeutically active agent" means any molecule that has or may have a therapeutic effect (i.e. curative or stabilizing effect) in the context of treatment of a disease (as described further herein).

Preferably, a therapeutically active agent is a disease-modifying agent, which can be a cytotoxic agent, such as a toxin, or a cytotoxic drug, or an enzyme capable of converting a prodrug into a cytotoxic drug, or a radionuclide, or a cytotoxic cell, or which can be a non-cytotoxic agent. Even more preferably, a therapeutically active agent has a curative effect on the disease.

By "treatment" is meant that at least an amelioration of the symptoms associated with the condition afflicting the host is achieved, where amelioration is used in a broad sense to refer to at least a reduction in the magnitude of a parameter, e.g. symptom, associated with the condition being treated. As such, treatment also includes situations where the pathological condition, or at least symptoms associated therewith, are completely inhibited, e.g., prevented from happening, or stopped, e.g. terminated, such that the host no longer suffers from the condition, or at least the symptoms that characterize the condition. Thus treatment includes: (i) prevention, that is, reducing the risk of development of clinical symptoms, including causing the clinical symptoms not to develop, e.g., preventing disease progression to a harmful state; (ii) inhibition, that is, arresting the development or further development of clinical symptoms, e.g., mitigating or completely inhibiting an active disease, e.g., so as to decrease tumor load, and/or to decrease the cancer metastases. Such treatment also includes situations where the pathological condition, or the progression of a pathological condition towards a more advanced disease state, or at least symptoms associated therewith, is reduced, or slowed down. In some cases, treatment includes situations wherein the mean time for survival between a patient population undergoing treatment comprising the administration of one or more uPAR-binding immunoglobulin single variable domains of the present disclosure and a control population not undergoing treatment is greater. In some cases, the increase in mean time for survival may be statistically significant.

A variety of hosts are treatable. Generally such hosts are "mammals" or "mammalian," where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In many embodiments, the hosts will be humans or canines, given the capability of the immunoglobulin single variable domains of the invention to specifically bind to human and canine urokinase plasminogen activator receptor.

The present invention will be now described in more details, referring to examples that are not limitative.

### EXAMPLES AND DESCRIPTION OF FIGURES

The study discussed in the following examples led to the development and preclinical validation of sdAbs that enable fast and highly specific *in vivo* imaging to visualize both human and canine uPAR expression through *in vivo* nuclear and fluorescence imaging techniques. In particular, the lead compound sdAb 15 (having SEQ ID NO: 2), recognizing human uPAR, is relevant towards future clinical translation, for example as contrast agent to predict non-invasively tumor aggressiveness or to guide surgical procedures in real-time. The capability of this sdAb to also target the canine homologue of uPAR opens the possibility to use this sdAb for veterinary applications. Moreover, it enables us to better assess its clinical potential making use of relevant large animal models with spontaneous tumors; this prior larger investments are made for clinical translation. Methods used in the examples are discussed in more detail below the examples.

### Example 1 : Generation and in vitro characterization of uPAR-specific sdAbs

First, a llama was immunized with both human (h) and murine (m) recombinant uPAR extracellular domain protein according to a standardized protocol. Following isolation of the peripheral blood B-lymphocytes and retrieval of the sequences coding for the affinity-matured V_{H}H gene regions, an immune library was created containing 7× 10⁸ individual transformants (library 161). uPAR-specific sdAbs were retrieved via phage display based on four rounds of panning on either h, m or canine (c) recombinant uPAR protein, yielding in total 150 binders as confirmed in a bacterial extraction ELISA (BE-ELISA). The best 37 clones (18 h, 15 m and 4 c uPAR binders, divided over 29 families that originate from the same genomic VDJ segment recombination) were screened for cross-reactivity amongst the different uPAR homologues in a follow-up BE-ELISA **(****Figure 1** **)** and their dissociation rate (k_{off}) was estimated via surface plasmon resonance (SPR) **(Table 1).** From these, 13 sdAbs (h and/or c binders) were selected and recloned into a pHEN6c expression vector (to link them with a carboxy-terminal hexahistidine tag) and transformed into WK6 *Escherichia coli (E. coli*) cells for IPTG-induced expression. Proteins were extracted via osmotic shock and further purified via affinity chromatography and size-exclusion chromatography. Production yield per clone is indicated in **Table 1**.

The thermal stability of the sdAbs was determined via a thermal shift assay. Melting temperatures (Tₘ) ranged between 59.5 and 73.6°C **(Table 1).** The kinetic parameters (kₐ, k_{d}, K_{D}) of the purified sdAbs were evaluated via SPR on respectively h and c uPAR recombinant protein **(****Figure 10****, Table 1 ).** Contrarily to the BE-ELISA results, no h/m or h/m/c cross-reactive sdAbs were identified. Only sdAbs 15-18 were found to be cross-reactive for h and c uPAR protein (SED ID NOs: 2 and 20-22, respectively). SdAbs with K_{D}-values < 10 nM were preferred; sdAbs with high k_{d}-values (≥ 1.5× 10⁻² 1/s) were unfavored. Finally, their binding to cell-membraneexpressed uPAR was investigated via flow cytometry using 1) h or c uPAR lentivirally transduced HEK293T cells and 2) U87 or HT29 cells with natural h expression. SdAbs showing a significant shift in binding compared to the control conditions (i.e., binding of the non-targeting sdAb R3B23) were further taken into consideration **(****Figure 2****).** Based on the combined results in terms of production yield, stability, binding characteristics, and cross-reactivity, 2 anti-h uPAR (sdAb 3 and 4) and 4 cross-reactive h/c uPAR (sdAb 15, 16, 17 and 18, having respectively SEQ ID NOs: 2, 20, 21, 22) sdAbs were selected for further *in vivo* evaluation.

**Table 1: Characteristics overview of the selected binders divided according to the targeted uPAR homologue (human, canine).**

| **Human binders** | | | | | | | |
|---|---|---|---|---|---|---|---|
| **Clone name** | **Nr** | **Prod. yield [mg/ l]** | **Tₘ [°C]** | **k_{off} [s^{- 1}](Hu)** | **kₐ [M⁻¹× s^{- 1}](Hu)** | **k_{d} [s^{- 1}](Hu)** | **K_{D} [M](Hu)** |
| **1 A4** | **1** | 1.85 | 64.8 | 5.1E-03 | 3.5E+05 | 6.2E-03 | 1.8E-08 |
| **1 A6** | **2** | 6.96 | 63.5 | 1.7E-01 | 1.7E+06 | 2.3E-01 | 1.4E-07 |
| **1 C8** | **3** | 3.34 | 73.6 | 8.9E-03 | 1.2E+06 | 7.5E-03 | 6.0E-09 |
| **1D8** | **4** | 4.14 | 60.4 | 4.3E-03 | 1.2E+06 | 5.2E-03 | 6.0E-09 |
| **2A4** | **5** | 0.53 | n/a | 6.2E-03 | 8.6E+05 | 7.3E-03 | 8.4E-09 |
| **2 C6** | **6** | 2.03 | n/a | 6.4E-03 | 4.6E+05 | 7.2E-03 | 1.6E-08 |
| **2C9** | **7** | 7.29 | 66.8 | 1.3E-03 | 1.1E+06 | 2.2E-01 | 2.0E-07 |
| **2D1** | **8** | 4.80 | 59.5 | 8.6E-04 | 6.1E+04 | 1.0E-03 | 1.7E-08 |
| **2D4** | **19** | 1.29 | 67.9 | 9.9E-03 | 4.3E+05 | 3.5E-03 | 8.2E-09 |
| **5A4** | **15** | 2.30 | 61.2 | 4.2E-03 | 1.6E+06 | 3.4E-03 | 2.1E-09 |
| **5A5** | **16** | 2.14 | 66.5 | 2.7E-03 | 5.0E+05 | 1.5E-03 | 3.0E-09 |
| **5B7** | **17** | 4.00 | 66.3 | 2.6E-03 | 8.6E+05 | 1.9E-03 | 2.3E-09 |
| **5H2** | **18** | 3.63 | 62.1 | 3.1E-03 | 8.4E+05 | 4.0E-03 | 4.7E-09 |

| **Canine binders** | | | | | | | |
|---|---|---|---|---|---|---|---|
| **Clone name** | **Nr** | **Prod. yield [mg/ l]** | **Tₘ [°C]** | k_{off} **[s^{- 1}](Cu)** | **kₐ [M⁻¹ × s^{- 1}](Cu)** | **k_{d} [s^{- 1}](Cu)** | **K_{D} [M](Cu)** |
| **5A4** | **15** | 2.30 | 61.2 | 6.5E-03 | 1.7E+05 | 2.0E-03 | 1.2E-08 |
| **5A5** | **16** | 2.14 | 66.5 | 1.0E-02 | 8.6E+04 | 3.1E-03 | 3.6E-08 |
| **5B7** | **17** | 4.00 | 66.3 | 7.3E-03 | 1.2E+05 | 1.9E-03 | 1.7E-08 |
| **5 H2** | **18** | 3.63 | 62.1 | 9.2E-03 | 1.0E+05 | 3.5E-03 | 3.4E-08 |

### Example 2: Evaluation of in vivo biodistribution and tumor targeting of uPAR-specific sdAbs via nuclear imaging

In a first *in vivo* experiment, the biodistribution and tumor targeting of the selected sdAbs were investigated via non-invasive imaging and *ex vivo* organ analysis in a xenografted Swiss nude mouse model bearing a subcutaneous (h or c) uPAR-expressing HEK tumor. Hereto, the sdAbs were radiolabeled with ^{99m}Tc on their carboxy-terminal hexahistidine tag via tricarbonyl chemistry and intravenously injected in the mice (n=4 per group). One-hour post-injection, mice were anesthetized, subjected to micro-SPECT/CT scanning, and then euthanized for organ collection. Representative SPECT/CT images are displayed in **Figure 3A****.** Uptake of the radiolabeled sdAbs in the organs and tissues of interest was quantified as percentage of injected activity per gram (%IA/g), corrected for radioactive decay **(****Figure 3B**-C, Tables 2 and 3). Adjusted P values were calculated based on the comparisons with control sdAb. Most h and c uPAR-binders present a typical biodistribution profile for sdAbs, with fast clearance from the blood (≤ 1.16 %IA/g at 1h post-injection), low uptake in normal organs and tissues, and high retention in the kidneys (ranging between 109±33 and 328±39 %IA/g). Contrarily, sdAbs 16 and 17 exhibit relatively high unspecific background signal, especially in blood (respectively 3.58±0.07 and 1.78±0.51 %IA/g) and liver (respectively 13.56±1.07 and 3.02±1.03 %IA/g). When further focusing on the tumor, sdAbs 15, 16 and 17, belonging to the same sdAb family, show significantly higher uptake than the non-targeting control sdAb R3B23 (0.20±0.08 %IA/g) in h uPAR HEK-tumors. Furthermore, sdAbs 15 and 17 show similar uptake in h (respectively 1.98±0.79 and 1.64±0.37 %IA/g) and c (respectively 1.46±0.03 and 1.70±0.31 %IA/g) uPAR expressing tumors. Although not significantly different, sdAb 4 exhibits also relatively good tumor uptake (1.28±0.27 %IA/g).

In a follow-up *in vivo* experiment, the biodistribution and tumor targeting potential of the most promising sdAbs (sdAb 4 and 15 as h and/or c binders) were evaluated in Swiss nude mice bearing a U87 xenograft. The cell line naturally expresses human uPAR on the surface of their cell membrane. The biodistribution of the h binders was comparable to the results of the previous experiments, although tumor uptake of sdAb 15 was higher and reached the value of 3.01±0.60 %IA/g **(****Figure 4A**-B, Table **4).** Adjusted P-values were calculated based on the comparisons with control sdAb. Based on all *in vivo* results, in combination with the previously obtained *in vitro* data, sdAb 15 (SEQ ID NO:2) was selected as final lead compound for h/c uPAR targeting

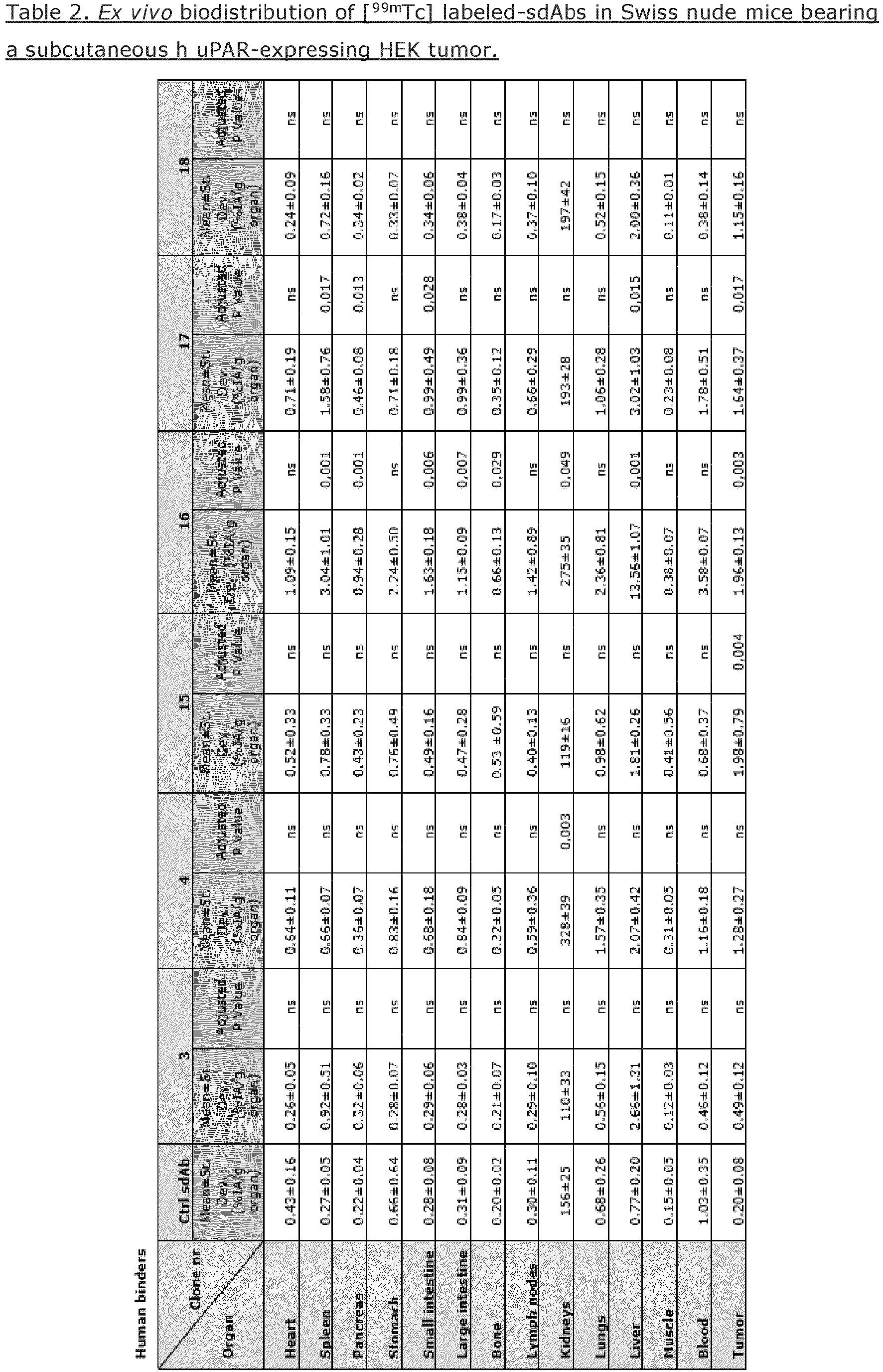

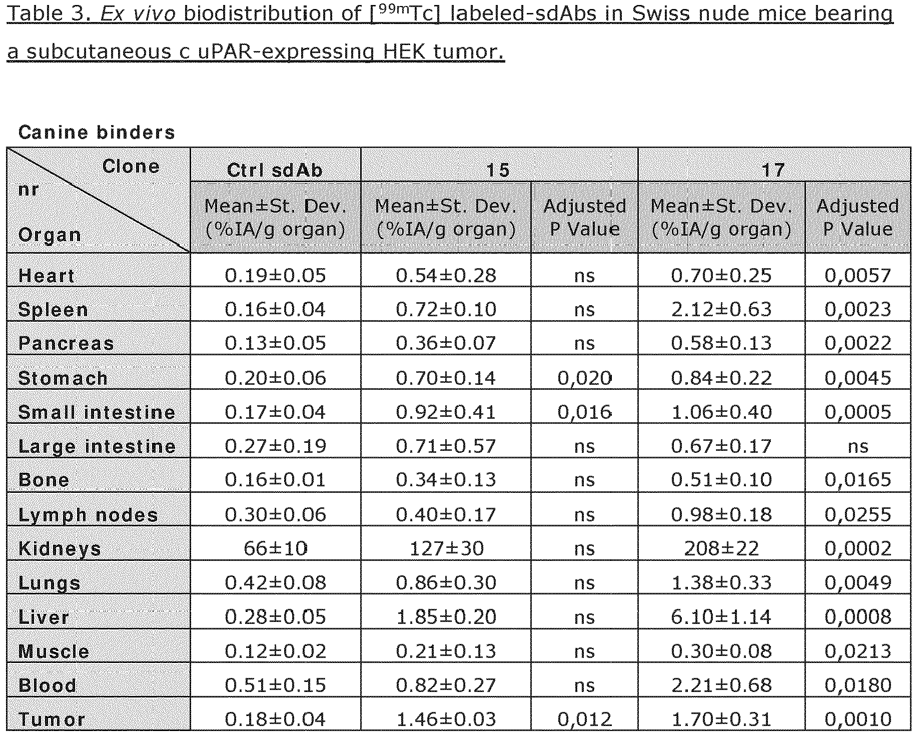

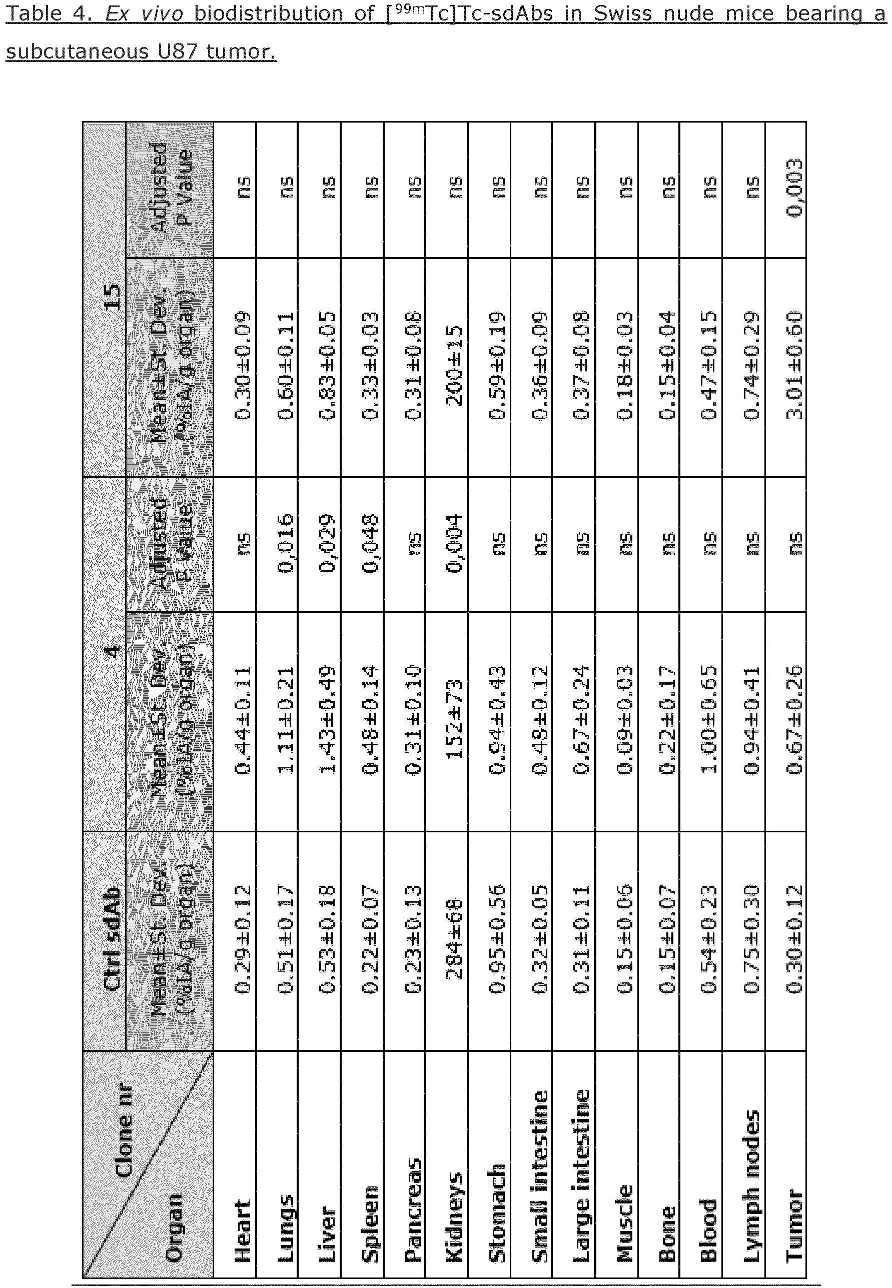

### Example 3: In vivo evaluation of fluorescently-labeled uPAR-specific sdAbs

The lead compound (sdAb 15) as identified in example 2 was subsequently labeled with the near-infrared fluorescent dye s775z via amine-reactive conjugation chemistry. The sdAb was incubated for 2 h at pH 8.5 with a 5-fold molar excess of NHS-ester activated s775z dye and purified via size-exclusion chromatography. A degree of labeling of 1 on average was obtained and spectral characteristics of the dye remained close to those of the free dye (ex/em maxima: 775/804 nm, **Figure 5A**-B). To confirm that fluorescent labeling of the selected sdAb did not impact the *in vivo* behavior as molecular tracer, the s775z-labeled sdAb 15 (2 nmol based on dye concentration) was intravenously injected into U87 -tumor bearing mice. Both *in vivo* images at 1h post-injection and *ex vivo* images of dissected organs show clear uptake of the tracers in the tumor with low background, except for the kidneys (renal clearance) **(****Figure 5C**-D). *Ex vivo* target-to-background ratios (TBRs, calculated with muscle as background) reached 8.55±3.12 for sdAb 15, which is considerably higher than for the control sdAb **(****Figure 6****).**

### Example 4: Proof-of-concept of intraoperative tumor imaging with fluorescently-labeled uPAR-specific sdAb

As proof-of-concept for the use of sdAb 15 as fluorescent tracer for image-guided surgery, the intraoperative imaging of brain cancer was mimicked in an orthotopic tumor model. U87 cells transduced with green fluorescent protein (GFP) and firefly luciferase (FLuc) were implanted in the right hemisphere of the brain (2 mm posterior and 2 mm lateral of the bregma) of Swiss nude mice. Tumor growth was monitored using bioluminescence imaging (10 min following intraperitoneal injection of 150 mg/kg luciferin). In parallel, a group of mice underwent sham surgery to control for a surge of uPAR expression due to wound healing at the implantation site. After approximately 3 weeks, 2 nmol of s775z-labeled sdAb 15 was injected intravenously and mice were killed 1 h post-injection (10 min prior killing, luciferase was also administered intraperitoneally to allow for subsequent *ex vivo* localization of the tumor using bioluminescence imaging, in addition to the imaging of the GFP signal). Two additional groups of tumor-bearing mice were administered either 2 nmol of the control sdAb R3B23-s775z or 2 nmol of sdAb15-s775z plus a 60x molar excess of unlabeled sdAb15. After removal of the skull, NIR fluorescent signals could clearly be observed in tumor-bearing mice injected with sdAb15-s775z using an intraoperative fluorescence imaging system (FluoBeam). Contrarily, no signal was detected in mice injected with the control sdAb, mice injected with an excess of unlabeled sdAb15 or in sham-operated mice. The fluorescent signal of sdAb15-s775z colocalized with the BLI and GFP signals **(****Figure 7A****).** *Ex vivo* TBRs, calculated using healthy brain tissue as background, reached 2.57±0.65 for sdAb15-s775z, and 0.99±0.39 or 1.34±0.09 for mice injected with excess of sdAb 15 or control sdAb, respectively. TBR for sham-operated mice was determined at 1.10±0.03. All the TBRs calculated for the control groups were significantly lower than the TBR of sdAb 15-s775z **(****Figure 7B****).**

### Example 5: In vivo evaluation of bimodal-labeled uPAR-specific sdAb

Finally, the lead anti-sdAb 15 was labeled site-specifically with both the near-infrared dye IRDye800CW and ^{99m}Tc to create a bimodal h uPAR-specific sdAb. sdAb 15 was genetically modified to carry a carboxy-terminal cysteine-tag separated by a 14 amino acid linker from the carboxy-terminal hexahistidine-tag. Maleimide-functionalized IRDye800CW was site-specifically attached to the cysteine-tag via thiol-reactive chemistry, and subsequently ^{99m}Tc was site-specifically labeled to the hexahistidine-tag via tricarbonyl-chemistry. After labeling the stability of the fluorescent signal of the bimodal sdAb 15 was assessed and radioactivity-dependence of the signal was established, with a significant decrease in or loss of fluorescent signal after labeling with ^{99m}Tc activities higher than 74 MBq **(****Figure 8A****).** The preserved fluorescent signals did stay stable over-time **(****Figure 8B****).** For further experiments, the ^{99m}Tc activity per mol of IRDye800CW-labeled sdAb at the start of labeling was set to 18.5 MBq/mol, resulting in bimodal sdAbs with well-preserved fluorescent signal for further experiments.

The effect of the bimodal label on the *in vivo* behavior and tumor targeting capacity of sdAb 15 was assessed. Bimodal-labeled sdAb 15 was intravenously injected in h uPAR⁺ HT-29-tumor bearing mice, and SPECT/CT and fluorescence imaging were performed 1, 4, 8 and 24 h post-injection. The *in vivo* SPECT/CT and fluorescent images indicated good biodistribution of the bimodal sdAb 15 without unexpected background signals, starting from 4 h post-injection **(****Figure 9A****).** *In vivo* TBRs, determined based on the *in vivo* fluorescence images, reached 2.4 ± 0.2 and 2.5 ± 0.1 for bimodal sdAb 15 at 4 h and 8 h post-injection, respectively, and were considerably increased compared to a bimodal labeled control sdAb R3B23 (1.6 ± 0.3 and 1.7 ± 0.3, respectively) **(****Figure 9B****).** *Ex vivo* analysis revealed a %ID/g of 1.1 ± 0.2 % for bimodal sdAb 15 in the tumor at 1 h post-injection, compared to 0.59 ± 0.09 % for the control bimodal sdAb **(****Figure 9C****).**

### Methods (Examples 1-5)

### Anti-uPAR sdAb generation

All sdAbs were generated and produced according to standard operating procedures, unless stated otherwise. Briefly, a llama was immunized with recombinant h or m uPAR antigen (Sino Biological, Beijing, P. R. China) according to a standardized vaccination scheme. After 6 weeks, B-lymphocytes were collected from peripheral blood and the mRNA of the heavy-chain Ab (HCAb) was extracted to generate cDNA using reverse transcriptase. Subsequently, the gene sequence encoding for the antigen binding fragment of the HCAbs was amplified by PCR. Next, the sdAb cDNA was ligated into a pMECS-GG vector in fusion with the phage gene III and transformed into E. *coli* cells to generate a library from which desired sdAbs can be further selected, according to the Golden Gate technology. Subsequently, biopannings were performed in parallel on immobilized h, m or c (produced by VIB, Ghent, Belgium) recombinant uPAR in four consecutive rounds. After each round, remaining clones were eluted and reamplified and colonies were picked randomly to prepare BEs. Briefly, randomly picked colonies were grown in 96-deep-well plates (Thermo Fischer Scientific, Waltham, MA, USA) in 1 mL 2xYT medium supplemented with D-glucose and ampicillin sodium (final concentrations 100 µg/mL and 0.1 %, respectively). At OD₆₀₀ = 0.6, expression was induced with 1 mM isopropyl β-d-1-thiogalactopyranoside (IPTG). After 4 h incubation at 37°C, plates were spun down, medium removed, and cells were lysed via overnight storage at -80°C. These extracts were then used in ELISA, and SPR off-rate screenings (k_{off}).

### ELI SA

96-well MaxiSorp plates (Thermo Fisher Scientific) were coated with 0.2 µg of h, m or c uPAR in 1 x PBS (pH 7.4, Gibco, Thermo Fisher Scientific) overnight at 4°C. The next day, wells were washed 5 times with 1x PBST buffer (1x PBS + 0.05% Tween 20), blocked with 2% skimmed milk in 1x PBS suspension for 1 h and washed again with 1x PBST. Subsequently, a solution of 20 µL of the tested BEs in 80 µL of 1x PBS were added to coated and uncoated (negative control) wells and incubated for 1 h at room temperature. SdAbs were detected with mouse anti-hemagglutinin-mAb (1:2000; clone 16B12, BioLegend, San Diego, CA, USA) and alkaline-phosphatase-conjugated anti-mouse-IgG mAb (1:2000; #7056, Bioké, Leiden, The Netherlands). All antibodies were diluted in 1x PBS and allowed to bind for 1h at room temperature. Between each incubation step, wells were washed 10 times with 1x PBST. For chemiluminescent detection, p-nitrophenyl phosphate (PNPP) substrate solution (2 mg/ml in AP buffer; 100 mM NaCl, 100 mM Trizma base, 50 mM MgCl₂ × 6H₂O) was added and the signal was measured at OD₄₀₅ using the VersaMax ELISA Microplate Reader (Molecular Devices, Downingtown, PA, USA) at 2, 10, 30 and 60 min timepoints. The binding was determined based on the signal ratio of the positive and negative wells.

### Anti-uPAR sdAb production

Anti-uPAR sdAb DNA fragments were recloned into a pHEN6 expression vector to introduce a carboxyterminal hexahistidine (6His) tag and sdAbs were produced in 1L WK6 *E*. *coli* cultures in Terrific Broth medium supplemented with ampicillin sodium, D-glucose and MgCl₂ (final concentrations 100 µg/mL, 0.1 % and 2 mM, respectively), each. SdAb expression was induced at OD₆₀₀ = 0.6 with 1 mM IPTG overnight at 28°C. Extracts containing the soluble sdAbs were collected from *E. coli* after osmotic shock treatment. Briefly, following the overnight sdAb expression in *E*. *coli,* bacterial cell pellets were harvested and resuspended in 12 mL TES buffer per pellet of 1 L culture. The mixture was incubated on ice for while shaking (200 RPM), and after 1h osmotic shock was performed using 24 mL of TES buffer diluted 4x per pellet. Next, MgCl₂ was added to a final concentration of 10 mM and following cell pellets centrifugation for 30 min at 11.325 g, supernatant was collected. SdAbs were further purified using immobilized metal affinity chromatography (IMAC) on Ni²⁺ beads (HisPur^{™} Ni-NTA Resin, Thermo Fischer Scientific) and gel filtration on Superdex^{™} 75 10/300GL column (Cytiva, Marlborough, MA, USA) in 1x PBS (PBS tablets, Calbiochem, Burlington, MA, USA). The control sdAb, R3B23, having no targeting capacity in healthy mice, was produced in the same way.

### Surface plasm on resonance

SPR was performed using a Biacore device (Biacore T200; Cytiva). First, the CM5 sensor chip (Biacore, Series S Sensor Chip CM5; Cytiva) was activated with 1-ethyl-3-(3-dimethylaminopropyl)carbodiimide/N-hydroxysuccinimide (EDC/NHS; Amine Coupling Kit; Cytiva). Subsequently, h, m or c uPAR recombinant proteins (5 µg/mL) were immobilized on separate chips at pH 5. The SPR measurements were done at 25°C in HEPES buffered saline (HBS; Cytiva) as a running buffer, with a flow rate of 30 µL/min. For off-rate screening, the contact time was set at 120 s, dissociation time at 600 s, and the runs were replicated 5 times. For screening of other kinetic parameters (kₐ, k_{d}, K_{D}), the sdAbs were injected consecutively in three-fold serial dilutions, from 150 to 0.62 nM. The association step was allowed for 180 s, the dissociation step for 600 s. For both studies, regeneration of 20 s at 30 µL/min using 0.1 M glycine at pH 2.5 followed by a stabilization period of 60 s, was performed. The rate kinetic constants were determined by mathematical fitting using the 1:1 binding with drift and RI2 model proposed by the BIACORE Evaluation Software (Cytiva), and the k_{d}/kₐ ratio was used to determine the equilibrium dissociation constant (K_{D}).

### Cell lines and culture conditions

All cells were obtained from the American Type Culture Collection (ATCC, Manassas, VA, USA) and cultured at 37°C, 95% O₂ and 5% CO₂. After reaching 80% confluency, they were split following dissociation with TrypLe Express Enzyme (1X) (Gibco). HEK293T cells were cultured in DMEM (Gibco), supplemented with 1 % penicillin/streptomycin (10,000 units/mL / 10,000 µg/mL, Gibco), 1 % L-glutamine (200 mM, 100X; Gibco) and fetal bovine serum (10% FBS, PAN-Biotech GmbH, Aidenbach, Germany). Mouse and human colorectal adenocarcinoma (respectively MC38 and HT29) and human glioblastoma (U87) cell lines were cultured in media (DMEM for MC38 and U87, and McCoy's 5A (Modified) Medium (Gibco) for HT29) supplemented as described above. Medium for MC38 and U87 cells was additionally enriched with 1 % non-essential amino acids (100X; Gibco) and 1 % sodium pyruvate (100 mM, 100X; Gibco). HEK293T cells were lentivirally transduced with h, m, or c uPAR; MC38 and U87 cells with Green Fluorescent Protein and/or Firefly Luciferase (indicated as GFP+ and FLuc+, respectively). Briefly, 10⁵ of cells were seeded per well in a 6-well plate (Thermo Fisher Scientific) and were subsequently transduced with lentiviral vectors encoding relevant constructs in complete medium overnight. The packaging plasmid pCMVΔR8.9 and VSV.G-encoding plasmid pMD.G were a gift from Dr. Trono (University of Geneva, Geneva, Switzerland). The transfer plasmid pHR' trip CMV SIN encoding h, m and c uPAR, or GFP and the transfer plasmid pDUAL encoding FLuc with puromycin selection gene were designed *in silico* and synthetized by Integrated DNA Technologies, Inc. (IDT, Leuven, Belgium). Lentiviral vector production has been described previously. These cells were later cultured in corresponding media as described above. The FLuc+ cells were selected using puromycin (final concentration 1 µg/ml). Following the transduction, cells were characterized with flow cytometry using BD FACSCelesta^{™}.

### Flow cytometry

Confluent cell cultures were scraped with a 39 cm Cell Scraper (VWR International B.V., Amsterdam, The Netherlands). Hereafter, 1.5× 10⁵ cells per well were added to a V-shaped 96-well plate (Greiner Bio-One, Frickenhausen, Germany). Cells were stained in ice cold FACS buffer (PBS, 0.5% BSA, 0.1% NaN₃) for 1 h with 1 µg/mL of corresponding PE-labeled (clone VIM5-phycoerythrin, BioLegend) antibody as a positive control, for h uPAR. In parallel, to test sdAb binding on membrane-associated uPAR, additional cells were incubated for 1 h with 17 nM of the selected h or c binding sdAbs and the non-targeting control sdAb R3B23, followed by 15 min staining with anti-His-PE (1:50 dilution, clone GG11-8F3.5.1, Miltenyi Biotec). Each incubation step was followed by two washing steps with cold 1x PBS. Finally, the cells were screened with the BD FACSCelesta^{™} (HTS plate reader) flow cytometer using the BD FACSDiva^{™} software. Results were analyzed using FlowJo software package (BD Biosciences, San Jose, CA, USA).

### Thermostability

SYPRO orange gel protein stain (5000x concentrate in DMSO, Life Technologies, Bleiswijk, The Netherlands) at the final concentration of 11x in PBS was added to a reaction mix with 0.2 µg/µL of each tested sdAb in PBS until a final volume of 40 µL. In parallel, a blank sample (reaction mix without sdAb) was prepared. 12.5 µL of the 40 µL was added to a 96-well in triplicates. The plate was run on a thermal cycler using following settings: Filter combination: 'FRET'; Program: melting curves, 1 cycle; Target: 25°C no acquisition: Target: 95°C continuous acquisition with 40 acquisitions per °C (ramp: 0.5°C/min): Target: 25°C no acquisition (ramp is then 2.5°C/s).

### ^{99m} Tc-labeling of sdAbs

1.5 mL of fresh ^{99m}TcO₄⁻-eluate from a ^{99m}Tc generator (Drytec, GE Healthcare, Chicago, IL, USA) (around 3700 MBq) was added to a commercial IsoLink kit (Mallinckrodt B.V., Petten, The Netherlands) to reduce TcO₄⁻ to ^{99m}Tc-tricarbonyl ([^{99m}TC(CO)₃(H₂O)₃]). After boiling for 20 min, 1N HCI (200 µL) was added to neutralize the solution and decompose any residual boranocarbonate. 500 µL of [^{99m}Tc(CO)₃(H₂O)₃] was added to 1 mg/mL solution of sdAb containing a carboxy-terminal 6His-tag (50 µL) and incubated for 90 min at 50°C. Hereafter, the solution was loaded on a Sephadex G25 disposable column (NAP-5, Cytiva) to remove the unreacted [^{99m}Tc(CO)₃(H₂O)₃]. The ^{99m}Tc-labeled sdAb was eluted with 1 mL PBS (0.1% Tween) and the purified sdAb was pressed through a Millex filter (0.22 µM; Merck Millipore, Darmstadt, Germany) to eliminate possible aggregates. A radiochemical purity of at least 95% is expected and the percentage (%) of radiochemical purity before and after purification was checked via instant thin layer chromatography (ITLC). 2 µL of ^{99m}Tc-labeled sdAb was placed on an ITLC strip (Aligent, Santa Clara, CA, USA) approximately 1 cm from the bottom. The bottom of the strip was put in 100% acetone, which was allowed to migrate to the top of the strip. The strip was cut in the middle and amount of radioactivity on both pieces was measured in a gamma-counter (Veenstra Dose Calibrator, Comecer, Joure, The Netherlands).

### Animal housing and husbandry

All animal experiments were conducted under approval of the ethical commission for animal experimentation of the Vrije Universiteit Brussel (projects 19-272-10, 20-272-13, 20-272-14 and 21-272-06). Swiss nude Crl:NU(Ico)-Foxn1nu were used (4 per group, 6-10 weeks of age; 20-30 g) and housed in individually ventilated cages (3-4 mice per cage) at 19-24°C in 40-60% humidity with a light/dark cycle of 14/10h. The number of mice allocated to each experiment was determined based on a prior power analysis and mice were randomly assigned to experimental and control groups. Animals had *ad libitum* access to water and, depending on the injection of fluorescent tracer during the experiment, normal (Safe, Rosenberg, Germany) or low fluorescence food pellets (Teklad 2016, Harlan Laboratories, IN, USA). Following the tumor cell inoculation, mice were visually checked every day for body condition score/body weight loss, physical appearance, and behavior. Mice were weighted and subcutaneous tumors were measured every 2-3 days with a digital caliper. A weight loss of ≥ 20% as compared to the moment of tumor inoculation, subcutaneous tumors with a size ≥ 1500 mm³, tumors with severe ulceration, or a severely infected surgical wound (intracranial tumor inoculation), or impacted mobility were considered humane endpoints. All mice were killed via cervical dislocation while either under isoflurane (5% for induction, 2% for maintenance and 1.5 L/min oxygen flow rate) or ketamine/xylazine (intraperitoneal injection, per kg: 100 mg/10 mg) anesthesia.

### In vivo biodistribution of ^{99m}Tc-labeled sdAbs

Depending on the experiment, different tumor cell types were inoculated (h or c uPAR transduced HEK293T cells (10⁷) or U87 cells (2× 10⁶)) under gas isoflurane anesthesia, in the right flank. After 2-4 weeks, subcutaneous tumors sized between 150-250 mm³ were grown. Equivalent of 30-60 MBq radiolabeled sdAb (∼5 µg) was injected via the tail vein in a volume of 150 µL. Subsequently, mice were anesthetized (intraperitoneal injection, per kg: 100 mg ketamine/10 mg xylazine, 10 min before imaging) and subjected to microSPECT/CT imaging at 1 h post-injection. Imaging was performed using a VECTOR+ system (MILabs B.V., Houten, the Netherlands) containing a general-purpose rat/mouse 1.5 mm 75 pinhole collimator. Spiral mode was used to take the scans with 6 bed positions (acquisition time of 200 s per bed position). Two subsets and four iterations were used for image reconstruction, with a voxel size of 0.4 mm in U-SPECT-Rec software (MILabs B.V.). The CT scan was made in 1 bed position, with a duration of 146 s at 60 kV and a pixel size of 80 µm. Image analysis was performed using a Medical Image Data Analysis Tool (AMIDE) and 3D images were reconstructed using OsiriX software (OsiriX MD; Pixmeo, Bernex, Switzerland).

After the SPECT/CT scan, mice were killed by cervical dislocation to undergo dissection and organ collection. The radioactivity in each organ and tissue of interested was counted by the gamma counter (2480 WIZARD, Perkin Elmer, Waltham, MA, USA) and expressed as percentage injected activity per gram of tissue (%IA/g), corrected for decay. The biodistribution of each sdAb was compared to that of the control sdAb.

### Fluorescent labeling of sdAbs

4 mg of each of the selected lead targeting sdAbs and the control sdAb containing a carboxy-terminal 6His-tag were separately concentrated into a volume of 500-700 µL using a Vivaspin 2 (5,000 MWCO HY, Sartorius; Stonehouse, UK) at 2.000 g. Each reaction mixture was pH-adjusted by the addition of 100 µL K₂HPO₄ (1 M, pH 8.5; VWR International B.V.) to reach a pH of 8.4-8.7. Subsequently, 5x molar excess of the fluorescent dye s775z (Fluoroprobes, Scottsdale, AZ, USA) was added to the mixtures for an incubation period of 2 h at room temperature. The purification of the labeled sdAbs was performed by size-exclusion chromatography (NGCTM Chromatography System, Bio-Rad; Hercules, CA, USA) using a Superdex^{™} 75 increase, 10/300 GL column (Cytiva) with 1x PBS as elution buffer at a flow rate of 0.6 mL/min. The labeled sdAbs were collected in different fractions by a BioFrac^{™} fraction collector (Bio-Rad). The concentration of the sdAbs (c_{sdAb}) and the dye (c_{dye}) were determined by measuring the absorbance at wavelengths 280 nm and 775 nm, respectively, using a UV-Vis spectrophotometer (NanoDrop^{™} 2000, Thermo Fisher Scientific) and the use of Lambert-Beer law. For the sdAb concentration, a correction factor of 3% was applied to correct for the absorbance of the dye at 280 nm. Finally, the degree of labeling (DOL) was determined by calculating the ratio c_{dye} over c_{sdAb}. A DOL around 1 was aimed for.

### Spectral characterization of fluorescently-labeled sdAbs

Samples of each fluorescent sdAb, corresponding to a dye concentration of 1 µM in 1 mL of PBS, and a reference PBS solution were transferred to quartz cuvettes. Excitation and emission (collected at maximal excitation wavelength, i.e., 775 nm) spectra for the samples were acquired separately using spectrofluorometry (Shimadzu RF-6000, Kyoyto, Japan), every 1 nm between 200 and 900 nm at a scan speed of 2000 nm/min. The data was normalized to the highest value. The values between 200 and 600 nm were at background level and were excluded from the graphs for clarity.

### In vivo biodistribution of fluorescently-labeled sdAbs

Swiss nude Crl:NU(Ico)-Foxn1nu bearing subcutaneous U87 tumors were intravenously injected with 100-200 µL containing 2 nmol, based on the concentration of the dye, of sdAb15-s775z and were compared to mice injected with non-targeting control R3B23-s775z. Subsequently, dorsal fluorescence imaging was performed 1 h post-injection on isoflurane-anesthetized mice using the NIR fluorescence camera Fluobeam^{®} (Fluoptics, Grenoble, France). Immediately after *in vivo* imaging, mice were killed, and organs of interest were collected for *ex vivo* imaging. Images were analyzed using ImageJ software. ROIs were determined based on the whole shapes of imaged tissues as seen on the white light images.

### Imaging of orthotopic brain tumor using fluorescently-labeled sdAb

Swiss nude Crl:NU(Ico)-Foxn1nu mice were placed in a stereotaxic frame (World Precision Instruments; Hertfordshire, UK) and an incision of 1-1.5 cm ranging from between the eyes to the base of the skull was made. Following the removal of the membrane on the skull with a scalpel, a microdrill was placed above the bregma and was repositioned 2 mm posterior and 2 mm lateral to the right to drill a hole through the skull. Next, 2.5× 10⁵ of GFP⁺/FLuc⁺ U87 cells resuspended in 5 µL or 5 µL PBS (sham surgery) were injected at a depth of 2.5 mm over a period of 5 min with a 10 µL Hamilton syringe (G26s, MICROLITERTM #701N; Hamilton, Reno, NV, USA). Finally, the hole in the skull was sealed off with bone wax and the skin was sutured. The tumor growth in the brain was monitored twice a week, 10 minutes after intraperitoneal injection of D-Luciferin (150 mg/kg; Promega; Leiden, The Netherlands), by bioluminescence imaging (PhotoIMAGER Optima, Biospacelab; Nesles-La-Vallée, France). Tumors were allowed to grow over a period of 3 weeks. Subsequently, mice were divided into four groups, i.e., mice bearing intracranial tumors injected intravenously with (i) sdAb-s775z, (ii) sdAb15-s775z 30 minutes after injection of 60x molar excess of unlabeled sdAb15, (iii) control sdAb R3B23-s775z and (iv) sham operated mice injected with sdAb15-s775z. A dose of 2 nmol, based on the concentration of the dye was injected per mouse. 1 h after injection of the labeled compound, mice were killed, craniectomy was performed, and fluorescence *ex vivo* imaging of the brain with Fluobeam was done. 10 min prior killing, D-Luciferin (150mg/kg) was intraperitoneally injected to enable *ex vivo* BLI imaging. In addition, GFP imaging was also performed using the PhotoIMAGER Optima. Image quantification was performed using ImageJ software (NIH, Bethesda, MD, USA). To quantify the fluorescence signal, ROIs were determined based on the GFP images or injection site for sham surgery. Corresponding ROIs were drawn on the healthy left hemisphere to acquire background signal.

### Bimodal labeling of sdAb

sdAb 15 and the control sdAb were genetically engineered to carry a carboxy-terminal tag consisting of a hexahistidine-tag, a 14 amino acid linker, and a cysteine tag, according to a previously described method. 3 mg of each sdAb was incubated with 90x molar excess of 2-MEA (ACROS organics, Thermo Fisher Scientific, Waltham, MA, USA) for the dimerized (or monomer) form of the sdAb and 5 mM EDTA (pH 8) (Sigma Aldrich, St. Louis, MO, USA) in 2.5 mL PBS (pH 7.4) for 90 min at 37 °C. Subsequently, the samples were applied to a PD-10 desalting columns (GE Healthcare, Chicago, IL, USA) pre-washed with 0.2 M ammonium acetate (Sigma-Aldrich, St. Louis, MO, USA) buffer (pH 6). The sample was eluted with 3.5 mL 0.2 M ammonium acetate buffer and the first 0.5mL was discarded. The 3 mL samples were transferred to a Vivaspin concentrators (Vivaspin 2, MWCO HY; Sartorius, Göttingen, Germany) and centrifuged at 3000 rpm for 15 - 45 min until a volume of 500 µL is obtained. The concentrated sdAbs were incubated with a 5x molar excess of IRDye800CW-maleimide (LI-COR Biosciences, Lincoln, NE, USA) and 5 mM EDTA for 120 min at 37°C, while being protected from light. The IRDye800CW-labeled sdAbs were purified via size-exclusion chromatography and the concentration and degree of labeling was determined using analogous protocols as described above. For the subsequent ^{99m}Tc-labeling, 2 nmol of the fluorescently-labeled sdAbs was incubated with 0, 37, 74, 148, 296 or 370 MBq (or 0, 18.5, 37, 74, 148 or 185 MBq/mol) ^{99m}Tc-tricarbonyl at 50°C for 90 min. Successful labeling was assessed via size-exclusion chromatography using a Superdex^{™} 75 increase 10/300 GL column with 1x PBS as elution buffer at a flow rate of 0.8 mL/min, with detection of the bimodal through both UV-VIS absorption at 774 nm and 280 nm, and gamma-ray detection. For *in vivo* experiments, 12 nmol of fluorescently-labeled sdAbs was incubated with 222 MBq ^{99m}Tc-tricarbonyl (18.5 MBq/mol) at 50°C for 90 min in a total volume of 500µL. Purification and purity control of the bimodal sdAbs was performed according to the same method as described above for ^{99m}Tc-labeling of sdAbs. The radiochemical purity had to be >95% for *in vivo* application of the bimodal sdAbs.

### In vivo evaluation of bimodal labeled sdAbs

Swiss nude Crl:NU(Ico)-Foxn1nu mice were subcutaneously inoculated with 2 × 10⁶ h uPAR⁺ HT-29 cells in the right flank and tumors were allowed to grow to 250-500 mm³. Mice were intravenously injected with 2 nmol ^{99m}Tc/IRdye800CW-labeled sdAb 15 or control sdAb R3B23, corresponding to 22 ± 2 MBq injected dose ^{99m}Tc per mouse. Mice were subjected to *In vivo* SPECT/CT imaging (MiLabs VECTOR⁺) and fluorescence imaging (Fluobeam) at 1, 4, and 8 h post-injection; at the 24 h time-point, only fluorescence imaging was performed because of the decay of the ^{99m}Tc. At the 1 h and 24 h post-injection time-point one group for each bimodal sdAb (n = 4/group) was killed to perform ex *vivo* analysis of the major organs via gamma counting (2480 WIZARD) and fluorescence imaging (Fluobeam). Inoculation, intravenous injection, and imaging were performed under isoflurane anesthesia (2% isoflurane, 1L O₂/min). *In vivo* and *ex vivo* fluorescent images were analyzed with ImageJ (Fiji). On the *in vivo* images, ROIs were drawn around the tumor and the contra-lateral muscle to allow *in vivo* TBR determination. On the ex vivo images, ROIs were drawn around all organs and tumor-to-organ ratios (TTOs) were determined. The *in vivo* SPECT/CT images were assessed qualitatively for unexpected or off-target background signals. The results from the *ex vivo* gamma counting were analyzed to determine the %ID/g for all excised tissues. The TBRs, TTOs, biodistribution, and %ID/g were compared between the bimodal sdAb 15 and the bimodal control sdAb.

### Statistical analyses

Statistical analyses on tumor uptake of radioactively/fluorescently-labeled sdAbs were performed using Prism software v.9.4.1. The radioactive sdAb uptake in tumor and organs of interest as compared to the control sdAb was analyzed using a Kruskal-Wallis test followed by a Dunn's multiple comparisons test. The fluorescently-labeled sdAbs with the associated MFI and TBR analyses in comparison to the control R3B23-s775z were performed using a Mann-Whitney test. Statistical significance was set at p<0.05 (^{∗}p<0.05, ^{∗∗}p<0.01, ^{∗∗∗}p<0.001, ^{∗∗∗∗}p<0.001). Descriptive statistics are displayed in the tables as mean ± standard deviation (St. Dev.).

The present invention is in no way limited to the embodiments described in the examples and/or shown in the figures. On the contrary, methods according to the present invention may be realized in many different ways without departing from the scope of the invention.

## Claims

1. An immunoglobulin single variable domain that is directed against and/or that specifically binds to human urokinase plasminogen activator receptor (human uPAR, SEQ ID NO: 1), wherein the immunoglobulin single variable domain comprises polypeptides that have at least 90% amino acid sequence identity with SEQ ID NO: 2.

2. The immunoglobulin single variable domain according to claim 1, wherein the immunoglobulin single variable domain comprises an amino acid sequence that comprises 4 framework regions (FR) and 3 complementarity determining regions (CDR) according to the following formula (1):
FR1-CDR1-FR2-CDR2-FR3-CDR3-FR4 (1);
and wherein the complementarity determining regions (CDRs) comprises polypeptides that have at least 90% amino acid sequence identity with SEQ ID NO: 3 (CDR1), SEQ ID NO: 4 (CDR2), SEQ ID NO: 5 (CDR3).

3. The immunoglobulin single variable domain according to claim 2, wherein the framework regions (FRs) have an amino acid sequence identity of more than 90% with the FRs of SEQ ID NO: 6 (FR1), SEQ ID NO: 7 (FR2), SEQ ID NO: 8 (FR3), SEQ ID NO: 9 (FR4).

4. The immunoglobulin single variable domain according to claim 2, wherein CDR1 consists of SEQ ID NO: 3, CDR2 consists of SEQ ID NO: 4, and CDR3 consists of SEQ ID NO: 5; and wherein the framework regions (FRs) have an amino acid sequence according to SEQ ID NO: 6 (FR1), SEQ ID N0: 7 (FR2), SEQ ID NO: 8 (FR3), SEQ ID NO: 9 (FR4).

5. The immunoglobulin single variable domain according to any of the previous claims, wherein the immunoglobulin single variable domain comprises an amino acid sequence according to SEQ ID NO: 2.

6. The immunoglobulin single variable domain according to any of the previous claims, wherein said immunoglobulin single variable domain is fused to a detectable label.

7. The immunoglobulin single variable domain according to claim 6, wherein said detectable label is a radionuclide and/or a fluorescent moiety.

8. The immunoglobulin single variable domain according to any of claims 1 to 7, wherein said immunoglobulin single variable domain is fused to a functional moiety.

9. The immunoglobulin single variable domain according to claim 8, wherein said functional moiety is a therapeutically active agent.

10. A polypeptide comprising an immunoglobulin single variable domain according to any of the previous claims 1-5.

11. A nucleic acid sequence encoding an immunoglobulin single variable domain according to any of the previous claims 1 - 5, or a polypeptide according to claim 10.

12. A pharmaceutical composition comprising the immunoglobulin single variable domain according to any of claims 1 to 9, or the polypeptide according to claim 10, and optionally at least one of a pharmaceutically acceptable carrier, adjuvant or diluent.

13. The immunoglobulin single variable domain according to any of claims 1 to 9, or the polypeptide according to claim 10, for use as contrast agent in non-invasive *in vivo* imaging.

14. The immunoglobulin single variable domain according to any of claims 1 to 9, or the polypeptide according to claim 10, for use in diagnosis, prognosis and/or treatment of cancer.

15. The immunoglobulin single variable domain according to any of claims 1 to 9, or the polypeptide according to claim 10, wherein the immunoglobulin single variable domain specifically targets human and/or canine uPAR-positive cells.
